# EUROPEAN PATENT SPECIFICATION

(11) **EP 2 576 472 B1**
(45) Date of publication and mention of the grant of the patent: **23.03.2016**
(21) Application number: 11727842.4
(22) Date of filing: 25.05.2011
(51) Int. Cl.: C03C 17/30, C03C 17/42, C08F 220/34, C09D 5/16, C09D 183/10, C08G 77/26, G06F 3/041

(54) **ANTIMICROBIAL COATINGS**
ANTIMIKROBIELLE BESCHICHTUNGEN
REVÊTEMENTS ANTIMICROBIENS

(30) Priority: 25.05.2010 US 348157 P; 25.05.2010 US 348044 P
(43) Date of publication of application: 10.04.2013
(73) Proprietor: 3M Innovative Properties Company, Saint Paul, MN 55133-3427 (US)
(72) Inventor: ALI, Mahfuza, B., Saint Paul, Minnesota 55133-3427 (US); JING, Naiyong, Saint Paul, Minnesota 55133-3427 (US); LIRINE, Valeri, Saint Paul, Minnesota 55133-3427 (US); NAGARKAR, Pradnya, V., Saint Paul, Minnesota 55133-3427 (US); YLITALO, Caroline, M., Saint Paul, Minnesota 55133-3427 (US); LENNHOFF, Nancy, S., Saint Paul, Minnesota 55133-3427 (US); STEPANOVA, Narina Y, Inver Grove Heights, MN 55076 (US)
(74) Representative: Herzog, Fiesser & Partner Patentanwälte PartG mbB
(86) International application number: PCT/US2011/037828
(87) International publication number: WO 2011/150001

(56) References cited:
- WO-A1-2010/036465
- US-A- 5 266 222
- US-A1- 2001 013 907
- US-A1- 2006 046 078
- US-A1- 2007 160 781

## Description

### CROSS REFERENCE TO RELATED APPLICATIONS

This application claims the benefit of U.S. Provisional Patent Application Nos. 61/348,044, filed on May 25, 2010.

### BACKGROUND

Surfaces that are intended to be touched by human operators, accordingly, will be exposed to the microorganisms either typically or incidentally found on skin. Touch panels, for example, can be found in applications from ATM's to casinos to point of sale terminals and portable computers. Since the data entry is based on contact, touch panels are inherently susceptible to scratches and to microbial contamination. Other surfaces that may be prone to contamination include countertops, bedrails, writing utensils, and keypads, for example.

US 2001/013907 A1 discloses an anti-microbial touch panel including a substrate, an active portion on one surface of the substrate and a homeotropic organosilane layer deposited on the active portion for reducing survivability of microorganisms contacting the touch panel.

US 5 266 222 A describes a formulation for forming a low surface-energy surface on a substrate having a fluoroalkyl silane having a low surface energy part, a liquid crystal silane operable for enhancing the orientation of the molecules of the fluoroalkyl silane and for cross-linking with the fluoroalkyl silane and a transport medium for applying the fluoroalkyl silane and the liquid crystal silane to the surface of a substrate.

WO 2010/036465 A1 discloses a polymeric material, methods of making the polymeric material, articles that include the polymeric material, and compositions that contain the polymeric material. The polymeric material has a plurality of different pendant groups that include a first pendant group containing a -COOH group or a salt thereof, a second pendant group containing a poly(alkylene oxide) group, a third pendant group containing a silicon-containing group, and a fourth pendant group containing a quaternary amino group. The polymeric material can be used to provide coatings that can be antifouling, antimicrobial, or both.

These surfaces provide a suitable home for bacteria, fungi, algae, and other one celled organisms which thrive and propagate based on the availability of appropriate amounts of moisture, temperature, nutrients, and receptive surfaces. As these organisms metabolize, they produce chemical by-products. These chemicals are known to damage (e.g., etch) certain surfaces (e.g., touch sensitive panels). Further, the biomass of such colonies fog or obscure the optical properties of the surfaces, irreparably damaging them. Cleaning and disinfection with chemicals which leach and poison the organisms and environmental controls which minimize moisture have, to date, been the response to this problem. Although cleaning and disinfection is common practice, it is done with the knowledge of the risks of sub-lethal dose levels, ineffective doses, resistant organisms, environmental exposure, human exposure, and the limited duration of such cleaners after the initial treatment.

Typical touch screen panels, e.g., capacitive touch screen panels, require direct contact with the skin of the user's finger. Thus, these panels are directly contacted by many different users. As these organisms thrive, the variety of chemicals that these organisms produce are also known to affect the human user. Thus, these microorganisms, as well as their metabolic products can pose serious health risks to users ranging from minor skin irritation to more serious toxic response and disease.

The foregoing concerns demonstrate growing detrimental effects of microorganisms on computer touch panels and a need for controlling microorganisms that may be disposed on such touch sensitive panels. The use of environmental controls has limited effectiveness on microorganism prevention in part because of the wide variety of environmental conditions under which various microorganisms can survive and in part because of the costs and difficulty of actually keeping moisture levels sufficiently low to minimize microbial growth.

There exists a need for simple means to prevent the colonization of articles by microorganisms and/or a means to reduce the number of living microorganisms that become disposed on a surface.

### SUMMARY

In view of the general need to control the number of viable microorganisms on a surface that is intentionally touched by its user, the present disclosure provides a composition as set out in claim 1 and an article as set out in claim 5. The antimicrobial polymer therein can be used, in some embodiments, to form a coating that is bonded to a touch-sensitive surface. The antimicrobial polymer may include chemical components that impart other desirable properties (e.g., adhesive properties, scratch resistance properties, antistatic properties) for the article on which it is applied. In some embodiments, the components of the polymer may be selected for their optically-transparent properties.

Thus, in one aspect, the present disclosure provides an article. The article can comprise a touch-sensitive substrate comprising a surface. The article further can comprise an organic polymer having a plurality of pendant groups. The organic polymer can be coupled to the surface. The plurality of pendant groups can comprise a first pendant group comprising a first quaternary ammonium component. The plurality of pendant groups further can comprise a second pendant group comprising a nonpolar component. The plurality of pendant groups further can comprise a third pendant group comprising a first organosilane or organic silane ester component. In some embodiments, the article further can comprise a siliceous substrate that includes a first side and a second side. In these embodiments, the organic polymer can be coupled to the first side of the siliceous substrate and the touch-sensitive substrate can be coupled to the second side of the siliceous substrate.

In any of the above embodiments, the article does not comprise a conductive layer.

In any of the above embodiments, the organic polymer further can comprise a second quaternary ammonium component.

In any of the above embodiments, the organic polymer further can comprise a second organosilane or organic silane ester component. In any of the above embodiments, at least one of the pendant components can comprise a fluorochemical.

In any of the above embodiments, in the organic polymer, the ratio of the number of N atoms associated with the first quaternary ammonium component and second quaternary ammonium component, if present, and the number of Si atoms associated with the first organosilane component and second organosilane component, if present, is about 0.1:1 to about 10:1.

In any of the above embodiments, the surface to which the organic polymer is coupled can be a glass or a polymeric surface. In any of the above embodiments, the siliceous substrate can be covalently coupled to the touch-sensitive substrate.

In another aspect, the present disclosure provides a method of making a coated article. The method can comprise forming a first composition of an organic polymer in a solvent. The polymer can have a plurality of pendant groups comprising: a first pendant group that includes a first quaternary ammonium component; a second pendant group that includes a nonpolar component; and a third pendant group that includes an organosilane or organic silane ester component. The method further can comprise mixing a second quaternary ammonium component with the first composition to form a first mixture. The method further can comprise contacting the first mixture with a substrate under conditions suitable to form covalent linkages between the organic polymer, the substrate, and the second quaternary ammonium compound. In some embodiments, the method further can comprise, after contacting the first mixture with the substrate, rinsing the coated article. In any of the above embodiments, the method further can comprise coupling the substrate to a touch-sensitive substrate. In any of the above embodiments, the method further can comprise providing a second composition comprising an adhesion-promoting reagent in a solvent and contacting the second composition with the substrate, wherein contacting the second composition with the substrate occurs prior to contacting the first mixture with the substrate.

In yet another aspect, the present disclosure provides a method of making a coated article. The method can comprise forming a first composition of an organic polymer in a solvent, mixing an adhesion-promoting reagent with the first composition to form a second mixture, and contacting the second mixture with a siliceous substrate under conditions suitable to form covalent linkages between organic polymer and the siliceous substrate. The polymer can have a plurality of pendant groups, including a first pendant group comprising a first quaternary ammonium component, a second pendant group comprising a nonpolar component, and a third pendant group comprising a first organosilane component. In any embodiment of the method, forming a second mixture further can comprise mixing a second quaternary ammonium component with the adhesion-promoting reagent and the first composition. In any embodiment of the method, the adhesion-promoting reagent can be selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(γ-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane. In any embodiment, the method further can comprise providing a second composition comprising an adhesion-promoting reagent in organic solvent and contacting the second composition with the substrate, wherein contacting the second composition with the substrate occurs prior to contacting the second mixture with the substrate.

In yet another aspect, the present disclosure provides a method of making a coated article. The method can comprise forming a first composition of an organic polymer in organic solvent. The polymer can have a plurality of pendant groups comprising a first pendant group that includes a first quaternary ammonium component a second pendant group that includes a nonpolar component, and a third pendant group that includes an organosilane or organic silane ester component. The method further can comprise mixing a second quaternary amonium compound with the first composition to form a first mixture. The method further can comprise contacting the first mixture with a touch-sensitive substrate under conditions suitable to form covalent linkages between organic polymer and the touch-sensitive substrate. In some embodiments, the method further can comprise, after contacting the first mixture with the touch-sensitive substrate, rinsing the coated article. In any of the above embodiments, the method further can comprise providing a second composition comprising an adhesion-promoting reagent in organic solvent and contacting the second composition with the touch-sensitive substrate, wherein contacting the second composition with the touch-sensitive substrate occurs prior to contacting the first mixture with the touch-sensitive substrate.

In yet another aspect, the present disclosure provides a method of making a coated article. The method can comprise forming a first composition of an organic polymer in organic solvent, mixing an adhesion-promoting reagent with the first composition to form a second mixture, and contacting the second mixture with a touch-sensitive substrate under conditions suitable to form covalent linkages between organic polymer and the touch-sensitive substrate. The polymer can have a plurality of pendant groups, including a first pendant group comprising a first quaternary ammonium component, a second pendant group comprising a nonpolar component, and a third pendant group comprising a first organosilane component. In any embodiment of the method, forming a second mixture further can comprise mixing a second quaternary ammonium component with the adhesion-promoting reagent and the first composition. In any embodiment of the method, the adhesion-promoting reagent can be selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyltrimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(y-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane. In any embodiment, the method further can comprise providing a second composition comprising an adhesion-promoting reagent in organic solvent and contacting the second composition with the touch-sensitive substrate, wherein contacting the second composition with the touch-sensitive substrate occurs prior to contacting the second mixture with the touch-sensitive substrate.

In yet another aspect, the present disclosure provides an antimicrobial polymer composition. The composition can comprise an organic polymer having a plurality of pendant groups with the proviso that the polymer does not comprise a pendant group that includes a carboxylate or alkoxylate chemical group. The pendant groups can include a first pendant group comprising a first quaternary ammonium component. The pendant groups further can include a second pendant group comprising a perfluorinated nonpolar component. The pendant groups further can include a third pendant group comprising a first organosilane component. In some embodiments, the antimicrobial composition further can include a fourth pendant component, wherein the fourth pendant component comprises a polar chemical group.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, "a," "an," "the," "at least one," and "one or more" are used interchangeably. Thus, for example, an article comprising "a" siliceous substrate can be interpreted to mean that the article can include "one or more" siliceous substrates.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### BRIEF DESCRIPTION OF THE DRAWINGS

The invention will be further explained with reference to the drawing figures listed below, where like structure is referenced by like numerals throughout the several views.
Figure 1a is a top perspective view of an embodiment of an antimicrobial touch screen article, with a capacitive layer, according to the present disclosure.
Figure 1b is a top perspective view of an embodiment of an antimicrobial touch screen article, without a capacitive layer, according to the present disclosure.
Figure 2 is a top perspective view of another embodiment of an antimicrobial touch screen article according to the present disclosure.
Figure 3 is a top perspective view of another embodiment of an antimicrobial touch screen article according to the present disclosure.
Figure 4 is a block diagram of one embodiment of a method of making a coated article according to the present disclosure.
Figure 5 is a bar graph showing the log reduction of *Staphylococcus aureus* bacteria according to one test method after exposure to several embodiments of an article comprising an antimicrobial polymer of the present disclosure.

### DETAILED DESCRIPTION

Polymeric materials are provided that can contain a plurality of different pendant groups. Methods of making the polymeric material and compositions that contain the polymeric material are also provided. Additionally, articles with coatings that contain the polymeric material are provided. The polymeric material in the coatings is often crosslinked. The coatings can be antimicrobial, scratch-resistant, or both.

Before any embodiments of the invention are explained in detail, it is to be understood that the invention is not limited in its application to the details of construction and the arrangement of components set forth in the following description or illustrated in the accompanying drawings. The invention is capable of other embodiments and of being practiced or of being carried out in various ways. Also, it is to be understood that the phraseology and terminology used herein is for the purpose of description and should not be regarded as limiting. The use of "including," "comprising," "containing," or "having" and variations thereof herein is meant to encompass the items listed thereafter and equivalents thereof as well as additional items. Unless specified or limited otherwise, the terms "supported," and "coupled" and variations thereof are used broadly and encompass both direct and indirect supports and couplings. It is to be understood that other embodiments may be utilized and structural or logical changes may be made without departing from the scope of the present disclosure. Furthermore, terms such as "front," "rear," "top," "bottom," and the like are only used to describe elements as they relate to one another, but are in no way meant to recite specific orientations of the apparatus, to indicate or imply necessary or required orientations of the apparatus, or to specify how the invention described herein will be used, mounted, displayed, or positioned in use.

The term "antimicrobial" refers to material that kills microorganisms or inhibits their growth.

The term "silane" refers to a compound having four groups attached to a silicon atom. That is, the silane has a silicon-containing group.

The term "alkoxysilyl" refers to a silicon-containing group having an alkoxy group bonded directly to the silicon atom. The alkoxysilyl can be, for example, of formula-Si(OR)(Rx)₂ where R is an alkyl and each Rx is independently a hydroxyl, alkoxy, alkyl, perfluoroalkyl, aryl, aralkyl, or part of a silicone.

The term "ester equivalent" means groups such as silane amides (RNR'Si), silane alkanoates (RC(O)OSi), Si-0-Si, SiN(R)-Si, SiSR and RCONR'Si that are thermally and/or catalytically displaceable by R"OH. R and R' are independently chosen and can include hydrogen, alkyl, arylalkyl, alkenyl, alkynyl, cycloalkyl, and substituted analogs such as alkoxyalkyl, aminoalkyl, and alkylaminoalkyl. R" may be the same as R and R' except it may not be H.

The term "hydroxysilyl" refers to a silicon-containing group having a hydroxyl group bonded directly to the silicon atom. The hydroxysilyl can be, for example, of formula - Si(OH)(Rx)₂ where Rx is an alkyl, perfluoroalkyl, aryl, aralkyl, alkoxy, hydroxyl, or part of a silicone. A compound having a hydroxysilyl group is often referred to as a "silanol". Silanols are a subset of silanes.

The term "silicone" refers to a moiety that contains a silicon-oxygen-silicon linkage group. Any other suitable groups can be attached to the silicon atoms. Such a linkage can result from the reaction of a first silane (e.g., a first silicon-containing group such as a first alkoxysilyl group or hydroxysilyl group) with a second silane (e.g., a second silicon-containing group such as a second alkoxysilyl group or hydroxysilyl group). In some embodiments, the silicone is part of a "silicone network". A silicone network results when a first silane (i.e., a first silicon-containing group) reacts with a second silane (e.g., a second silicon-containing group) plus a third silane (e.g., a third silicon-containing group such as a third alkoxysilyl group or hydroxysilyl group) or when a first silane (e.g., a first silicon-containing group) reacts with a second silane (e.g., a second silicon-containing group) plus a third silane (e.g., a third silicon-containing group) and a fourth silane (e.g., a fourth silicon-containing group such as a fourth alkoxysilyl group or hydroxysilyl group).

As used herein, the phrases "polymeric material with a plurality of pendant groups", "polymeric material with multiple pendant groups", or similar phrases are used interchangeably to refer to a polymeric material that has at least three different types of pendant groups. The multiple pendant groups include (1) a first pendant group containing a quaternary amino group; (2) a second pendant group containing a nonpolar group; and (3) a third pendant group having an organosilane group. The polymeric material with multiple pendant groups can be crosslinked through a condensation reaction of multiple organosilane groups. Furthermore, the polymeric material can be covalently coupled to a surface comprising a silanol group or, preferably, a plurality of silanol groups.

The present disclosure is generally directed to articles comprising an antimicrobial coating and methods of making said articles comprising an antimicrobial coating. The articles further comprise a substrate. In some embodiments, the substrate comprises a touch-sensitive substrate (e.g., a computer display touch panel). The touch-sensitive substrate may comprise an active portion. The active portion of the substrate includes a surface configured to be touched (e.g., by a finger, a stylus, or the like). "Active portion" is used herein in the broadest sense and refers to a region of the substrate that can transduce a tactile stimulus into an electrical signal. Nonlimiting examples of devices that comprise a substrate with an "active portion" include touch screens (e.g., computer touch screens, personal digital assistant touch screens, telephone touch screens, card reader touch screens, casino gaming devices, touch-enabled industrial equipment controls, touch-enabled vehicle accessory controls, and the like) Exemplary touch screens are disclosed in U.S. Patent Nos. 6,504,582; 6,504,583; 7,157,649; and U.S. Patent Application Publication No. 2005/0259378.

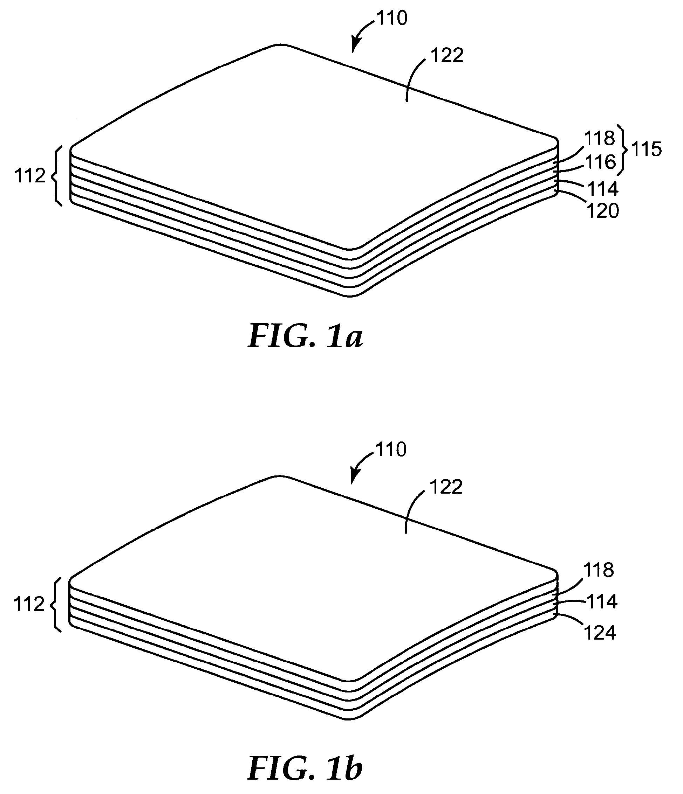

Turning to the Figures, FIG. 1a shows one embodiment of an antimicrobial touch sensor 110 according to the present invention. The touch sensor 110 may be a touch sensitive panel such as, for example, a "surface capacitive" computer touch panel, available from 3M Touch Systems, Methuen, Massachusetts, made up of several different layers. The touch sensor 110 features an antimicrobial touch panel 112.

Touch panel 112 includes electrically insulative substrate 114. Insulative substrate 114 may be constructed from glass, plastic or another transparent medium, for example. The touch panel 112 further comprises a touch-sensitive active portion 115 on the insulative substrate 114. Active portion 115 includes a transparent, electrically conductive layer 116 deposited directly on substrate 114. Conductive layer 116, for example, can be a tin oxide layer having a thickness of twenty to sixty nanometers and may be deposited by sputtering, vacuum deposition and other techniques known in the art. The thickness of the layers is exaggerated in FIG. 1a for illustrative purposes only and is not intended to represent the layers to scale. Conductive layer 116 may also include a conductive polymeric material or a conductive organic-inorganic composite.

A conductive pattern, not shown, can be disposed about the perimeter of conductive layer 116 to provide a uniform electric field throughout the layer 116 in order to establish the point of contact between the panel 112 and a finger or stylus.

Active portion 115 may also include protective layer 118 deposited over conductive layer 116 to provide abrasion resistance to protect conductive layer 116. Protective layer 118 may be a layer of an organosiloxane formed by applying to the article a composition (e.g., a solution) comprising methyltriethoxysilane, tetraethylorthosilicate, isopropanol and water. Additionally, or alternatively, the protective layer may comprise a hardcoat material (e.g., the glare-resistant hardcoat described in Example 1 of U.S. Patent No. 7,294,405).

Second conductive layer 120 may be provided to shield touch sensor 110 from noise which may result from the electric circuits of a display unit, not shown, to which display 110 may be attached and may similarly include a tin oxide layer deposited in a similar manner as discussed with reference to conductive layer 116. However, conductive layer 120 is not a necessary limitation of the invention as touch sensor 110 can function without it.

Antimicrobial polymer layer 122 in accordance with this disclosure is coupled to active portion 115, usually on protective layer 118 or even directly to conductive layer 116 if protective layer 118 is not present or to the outermost layer, if additional layers (not shown) are present to reduce energy dissipation of an object contacting touch sensor 110. In this configuration, antimicrobial polymer layer 122 can minimize or prevent damage to touch sensor 110, providing an easy glide experience to the touch screen user, as well as inhibit the survival and growth of microorganisms which come to rest on touch sensor 110.

FIG. 1b shows one embodiment of another antimicrobial touch sensor 110 according to the present invention. The touch sensor 110 may be a touch sensitive panel such as, for example, a "projected capacitive" computer touch panel in a projected capacitive touch screen, made up of several different layers. The touch sensor 110 features an antimicrobial touch panel 112.

Touch panel 112 includes electrically insulative substrate 114. Insulative substrate 114 may be constructed from glass, plastic or another transparent medium, for example.

A conductive layer 124 can be disposed beneath the substrate 114 in order to establish the point of contact between the panel 112 and a finger or stylus. In some embodiments (not shown), conductive layer 124 may be a plurality of conductive layers (e.g. arrays of electrodes) with dielectric layers disposed there between. Touch sensors with this configuration are disclosed in U.S. Patent Application No. 12/652,343.

Touch panel 112 may also include protective layer 118 deposited over insulative substrate 114 to provide abrasion resistance to protect insulative substrate 114. Protective layer 118 may be a layer of an organosiloxane formed by applying to the article a composition (e.g., a solution) comprising methyltriethoxysilane, tetraethylorthosilicate, isopropanol and water. Additionally, or alternatively, the protective layer may comprise a hardcoat material (e.g., the glare-resistant hardcoat described in Example 1 of U.S. Patent No. 7,294,405).

Antimicrobial polymer layer 122 in accordance with this disclosure is coupled to protective layer 118 or even directly to insulative substrate 114 if protective layer 118 is not present or to the outermost layer, if additional layers (not shown) are present to reduce energy dissipation of an object contacting touch sensor 110. In this configuration, antimicrobial polymer layer 122 can minimize or prevent damage to touch sensor 110, providing an easy glide experience to the touch screen user, as well as inhibit the survival and growth of microorganisms which come to rest on touch sensor 110.

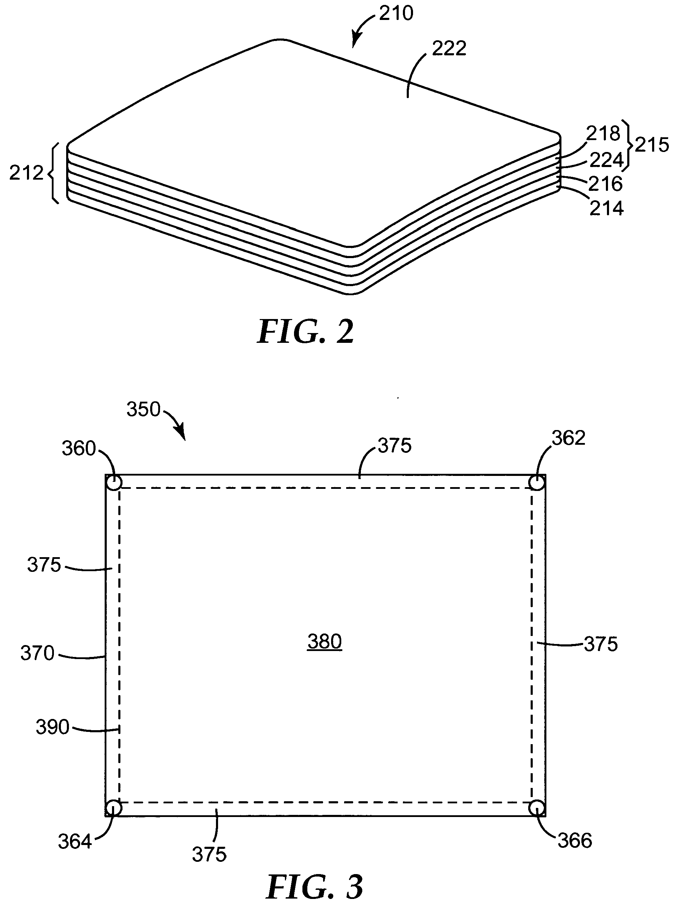

FIG. 2 shows another embodiment of a touch sensor 210. The touch sensor 210 may include, for example, a resistive computer touch panel 212, available from Elo TouchSystems, Freemont, Calif., which includes insulative substrate 214 and conductive layer 216, similar to FIG. 1a. Protective layer 218 may include a hard coating which protects and supports deformable conductive layer 224 interposed between conductive layer 216 and protective layer 218. A nonlimiting example of a suitable hard coating includes the glare-resistant hardcoat described in Example 1 of U.S. Patent No. 7,294,405. As touch sensor 210 is contacted by a finger or stylus deformable conductive layer 224 compresses and makes contact with conductive layer 216 to indicate the position of the contact. Antimicrobial polymer layer 222 is applied to protective layer 218.

FIG. 3 shows one embodiment of a vibration-sensing touch sensor 350 that includes a rectangular touch plate 370 and vibration sensors 360, 362, 364, and 366 located at the corners and coupled to the touch plate. When integrated into a system, for example overlaying an electronic display, the border portion 375 of touch sensor 350 may be covered by a bezel, leaving an intended touch area 380 exposed to a user. Dashed line 390 is used to indicate a separation between the border area 375 and the intended touch area 380. Dashed line 390 is an arbitrary designator, and does not necessarily indicate that touches outside of its inscribed area cannot be detected. To the contrary, dashed line 390 merely inscribes an area where touch inputs are intended or expected to occur, which may include the entire touch plate or some portion or portions thereof. When dashed lines are used in this document to designate intended touch areas, they are used in this manner. Antimicrobial polymers (not shown) of the present disclosure can be applied directly or indirectly to the surface of the touch area 380 of the vibration-sensing touch sensor 350. An example of indirect application includes applying the antimicrobial polymer to one side of a polymer film and a pressure-sensitive adhesive to the other side of the film; then applying the adhesive side of the film to the touch area of the vibration-sensing touch sensor.

While the touch plate is shown as rectangular in FIG. 3, it can be of any arbitrary shape. The touch plate can be glass, acrylic, polycarbonate, metal, wood, or any other material cable of propagating vibrations that can be caused or altered by a touch input to the touch plate and that can be sensed by the vibration sensors. To detect the touch position in two dimensions on the touch plate, at least three vibrations sensors can be used, and are generally located at peripheral portions of the touch plate, although other locations can be used. For convenience, redundancy, or other reasons, it may be desirable to use at least four vibration sensors, for example one at each corner of a rectangular touch plate, as shown in FIG. 3. The vibration sensors can be any sensors capable of detecting vibrations in the touch plate that are caused or affected by a touch, for example bending wave vibrations.

Piezoelectric materials may provide exemplary vibrations sensors. The vibration sensors can be mechanically coupled to the touch plate by use of an adhesive, solder, or other suitable material. Conductive traces or wires (not shown) can be connected to each of the vibration sensors for communication with controller electronics (not shown). Exemplary vibration-sensing touch sensors, their operation, their components, and their layout on a sensor are disclosed in co-assigned U.S. Patent Application Publication No. 2004/0233174 and U.S. Patent Application Publication No. 2005/0134574.

### Antimicrobial Polymers:

The present disclosure provides antimicrobial polymers. The antimicrobial polymers are formed by reacting, in suitable organic solvent, monomers that comprise a chemical group that serves one or more functional purposes in the polymer.

In some embodiments, the antimicrobial polymers can be coated (e.g., as a film or layer) onto a substrate as described herein. The polymers have antimicrobial activity that can kill or inhibit microorganisms that come into contact with the polymer (e.g., on the surface of a touch panel). The antimicrobial activity can be tested using a standardized antimicrobial resistance test such as, for example, JIS-Z 2801 (Japanese Industrial Standards; Japanese Standards Association; Tokyo, Japan). The polymers further have scratch-resistant properties. The scratch-resistant property of the polymer can be tested using the ASTM test method D 7027.26676.

Polymers of the present disclosure are formed in any suitable solvent (e.g., an organic solvent) that will solublize or make a dispersion of the resultant polymer. Suitable organic solvents have a boiling point about 200 °C or lower and can be mixed with small portions (<10%, w/w) of acidified water without substantially degrading the solvent properties. Adding the acidified water to the solvent facilitates complete hydrolysis of silane groups which, in turn optimizes the formation of -Si-O-Si- bonds within the polymer and between the polymer and the substrate. This can result in improved durability of antimicrobial coating on the substrate. Preferably, the solvent flashpoint is 100 °C or lower. Nonlimiting examples of suitable organic solvents include an alcohol (e.g., isopropyl alcohol, methanol), MEK, acetone, DMF, DMAC (dimethyl acetamide,) ethyl acetate, THF, etc. The monomers are mixed with the solvent and reacted to form an antimicrobial polymer. Suitable monomers include derivatives of acrylate monomers, methacrylate monomers, vinyl monomers, and olefinic monomers. The monomers comprise chemical groups that are pendant from the polymer after the polymerization reaction. The pendant groups include a first quaternary ammonium group, a nonpolar group, and a first organosilane group (e.g., trimethoxysilylpropane).

The polymer shown in Structure (I) shows a representation of a portion of an antimicrobial polymer made from acrylate or olefinic monomers according to the present disclosure.

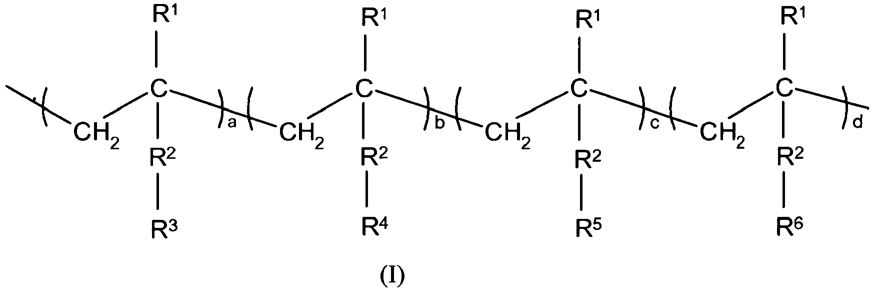

Polymers of the present disclosure include pendant groups with antimicrobial activity. The groups with antimicrobial activity can be selected for properties that are desirable in the articles on which the polymer is coated. For example, the antimicrobial group can be selected because it provides a polymer having substantial optical clarity (i.e. high optical transmission throughout a narrow or broad spectrum of wavelengths, low haze). These properties easily can be measured by a person of ordinary skill in the art, for example, by methods disclosed herein. In the exemplary polymer of Structure (I), the first quaternary ammonium pendant group includes the quaternary ammonium moiety R³ and can be derived from a monomer where:
R¹ = H or CH₃,
R² = COO, CO, C₁-C₁₂ alkyl, aryl
R³ = a quaternary ammonium having the formula
-(CH₂)ₙ-N(R⁷)(R⁸)(R⁹)(X⁻) where
   n = 1-3 (i.e., an alkyl group from C₁-C₃,)
   R⁷, R⁸, and R⁹ are independently an alkyl (C₁-C₂₂), aryl, or a combination of chemical groups forming a ring structure; and
   X = Cl, Br, N(SO₂CF₃)₂, BF₄, OSO₂C₄F₉, OSO₂CF₃, OSO₃CH₃.

The first quaternary ammonium pendant groups are coupled (e.g., covalently coupled) to the polymer such that, the antibiotic activity of the antimicrobial coupled to the polymer is insoluble in water (i.e., the antimicrobial is non-leaching when the polymer is contacted with an aqueous solution). Nonlimiting examples of suitable antimicrobial quaternary ammonium components include the hexadecyldimethylethylamine, octadecyldimethylethylamine, hexadecyldimethylpropylamine and octadecyldimethylpropylamine.

In the exemplary polymer of Structure (I), the nonpolar pendant group includes the nonpolar moiety R⁴ and can be derived from a monomer where R⁴ is an unsubstituted or substituted alkyl group (C₄ to C₂₂), an aryl group, perfluoroalkyl sulfonamide, perfluoroalkyl sulfone, perfluoroalkyl carboxamide, a class of free-radically reactive fluoroalkyl or fluoroalkylene group-containing compatibilizers of the respective chemical formulas: R_{ff}Q₃(X₁)ₙ₁ and (X₁)ₙ₁Q₃R_{ff2}Q₃(X₁)ₙ₁), where R_{ff} is a fluoroalkyl, R_{ff2} is a fluoroalkylene, Q₃ is a connecting group of valency at least 2 and is selected from the group consisting of a covalent bond, an alkylene, an arylene, an aralkylene, an alkarylene group, a straight or branched chain or cycle-containing connecting group optionally containing heteroatoms such as O, N, and S and optionally a heteroatom-containing functional group such as carbonyl or sulfonyl, and combinations thereof; X₁ is a free-radically reactive group selected from (meth)acryl, -SH, allyl, or vinyl groups and n1 is independently 1 to 3. Typical Q₃ groups include: -SO₂N(R)CH₂CH₂-; -SO₂N(CH₂CH₂)₂-; -(CH₂)ₘ-; -CH₂O(CH₂)₃-; and -C(O)NRCH₂CH₂-, where R is H or lower alkyl of 1 to 4 carbon atoms and m is 1 to 6. Preferably the fluoroalkyl or fluoroalkylene group is a perfluoroalkyl or perfluoroalkylene group. Exemplary, non-limiting perfluorobutyl-substituted acrylate compatibilizers meeting these criteria and useful in the present invention include one or more of C₄F₉SO₂N(CH₃)CH₂CH₂OC(O)CH=CH₂, C₄F₉SO₂N(CH₂CH₂OC(O)CH=CH₂)₂, or C₄F₉SO₂N(CH₃)CH₂CH₂OC(O)C(CH₃)=CH₂. One non-limiting example of a preferred fluoroalkyl-substituted monomers that may be utilized in the composition of the coat layer is: (1H,1H,2H,2H)-perfluorodecyl acrylate, available from Lancaster Synthesis of Windham, New Hampshire. Numerous other (meth)acryl compounds with perfluoroalkyl moieties that may also be utilized in the composition of the coat layer are mentioned in U.S. Patent No. 4,968,116, to Hulme-Lowe et al., and in U.S. Patent No. 5,239,026 (including perfluorocyclohexylmethyl methacrylate), to Babirad et al. Other fluorochemical (meth)acrylates that meet these criteria and may be utilized include, for example, 2,2,3,3,4,4,5,5-octafluorohexanediol diacrylate and *ω* -hydro 2,2,3,3,4,4,5,5-octafluoropentyl acrylate (H-C₄F₈-CH₂O-C(O)-CH=CH₂). Other fluorochemical (meth)acrylates that may be used alone, or as mixtures, are described in U.S. Patent No. 6,238,798, to Kang et al.

Another monomer that may be used is a fluoroalkyl- or fluoroalkylene-substituted thiol or polythiol. Non-limiting examples of this type of monomers includes one or more of the following: C₄F₉SO₂N(CH₃)CH₂CH₂OC(O)CH₂SH, C₄F₉SO₂N(CH₃)CH₂CH₂OC(O)CH₂CH₂SH, C₄F₉SO₂N(CH₃)CH₂CH₂SH, and C₄F₉SO₂N(CH₃)CH(OC(O)CH₂SH)CH₂OC(O)CH₂SH.

In another preferred embodiment, the coating composition adds one or more multi-olefinic compounds bearing at least one monovalent poly(hexafluoropropylene oxide) (HFPO) moiety and optionally a compatibilizer such as a fluoroalkyl- or fluoroalkylene-substituted mono or multi-acrylate such as C₄F₉SO₂N(CH₃)CH₂CH₂OC(O)CH=CH₂, C₄F₉SO₂N(CH₂CH₂OC(O)CH=CH₂)₂, or C₄F₉SO₂N(CH₃)CH₂CH₂OC(O)C(CH₃)=CH₂, alcohol, olefin, thiol or polythiol to fluoropolymer curing composition. Non-limiting examples of thiol or polythiol type of compatibilizer includes one or more of the following: C₄F₉SO₂N(CH₃)CH₂CH₂OC(O)CH₂SH, C₄F₉SO₂N(CH₃)CH₂CH₂OC(O)CH₂CH₂SH, C₄F₉SO₂N(CH₃)CH₂CH₂SH, and C₄F₉SO₂N(CH₃)CH(OC(O)CH₂SH)CH₂OC(O)CH₂SH.

As used in the examples, unless otherwise noted, "HFPO-" refers to the end group F(CF(CF₃)CF₂O)ₐCF(CF₃)- of the methyl ester F(CF(CF₃)CF₂O)ₐCF(CF₃)C(O)OCH₃, wherein "a" averages about 6.8, and the methyl ester has an average molecular weight of 1,211 g/mol, and which can be prepared according to the method reported in U.S. Patent No. 3,250,808 (Moore et al.) with purification by fractional distillation.

The mono- or multi-olefinic compound bearing at least one monovalent poly(hexafluoropropylene oxide) (HFPO) moiety preferably is in the form of a multiacrylate. These materials are of the formula: R_{fpe}Q(X)ₙ wherein Rfpe is the residue of a monovalent HFPO moiety, Q is a connecting group comprising an alkylene, arylene, arylene-alkylene, or alkylene-arylene group and may comprise a straight or branched chain connecting group which may contain heteroatoms such as O,N, and S, X is a free-radically reactive group selected from meth(acryl), allyl, or vinyl groups and n is 2 to 3. Typical Q group include: -(CH₂)ₘ-; -CH₂O(CH₂)₃-; and -C(O)NRCH₂CH₂-, where R is H or lower alkyl of 1 to 4 carbon atoms and m is 1 to 6.

One class of multi- (meth)acryl compound bearing at least one monovalent poly(hexafluoropropylene oxide) (HFPO) moiety comprises compounds described in U.S. Provisional Application No. 60/569,351 (Docket No. 59795US002) entitled "Fluoropolyether Polyacryl Compounds", filed May 7, 2004.

Other mono- and multi- (meth)acryl compounds bearing at least one monovalent poly(hexafluoropropylene oxide) (HFPO) moiety comprise compounds which are Michael adducts of HFPO amine derivatives with multiacrylates described in U.S. Application No. 10/841,792, entitled "Polymerizable Compositions, Methods Of Making The Same, And Composite Articles Therefrom," filed May 7, 2004.

The nonpolar pendant group is a chemical group that increases the relative hydrophobicity of the antimicrobial polymer. The nonpolar pendant groups are selected for their ability to influence the surface energy of the polymer. In particular, the nonpolar pendant groups are selected to impart a low surface energy polymer. Nonpolar pendant groups can also increase the scratch resistance of the polymer, when the polymer is coated onto a hard surface (e.g., glass). Nonlimiting example of suitable nonpolar groups include linear or branched alkanes (e.g., isooctane, isobutane) and aromatic groups.

In the exemplary polymer of Structure (I), the first organosilane pendant group includes the siloxane moiety R⁵ and can be derived from a monomer where:
R⁵ = (CH₂)ₘ-Si(OR¹⁰)₃,
m = 1-6 (i.e., an alkyl group from C₁-C₆,) and
   R¹⁰ = an alkyl group from C₁-C₃.

The first organosilane pendant group includes a silicon-containing group. This pendant group can crosslink the antimicrobial polymeric material, bond the antimicrobial polymeric material to a substrate, bond a second organosilane to the antimicrobial polymer, or it can confer the ability of the polymer to perform any combination of the foregoing bonding configurations. A nonlimiting example of a suitable organosilane pendant group is the propyl trimethoxysilane group found in methacryloylpropyl trimethoxysilane.

Although Structure (I) shows a portion of an exemplary antimicrobial polymer comprising three sequential monomers with different pendant groups, it will be recognized that the antimicrobial polymer of the present disclosure is a random copolymer, with the number and order of monomeric subunits (a, b, c, and, optionally, d) influenced by the respective ratios of monomeric units in the polymerization reaction and/or the polymerization reaction conditions.

Antimicrobial polymers of the present disclosure optionally can include, in addition to the quaternary ammonium, nonpolar, and organosilane pendant groups, a fourth pendant group that includes a polar component. The polar pendant group can confer adhesive properties that allow the antimicrobial polymer to adhere to certain substrates. Because the polar pendant groups promote adhesion of the antimicrobial polymer to the substrate, advantageously, this can result in an improved durability of the polymer on the substrate. In some embodiments, the polar pendant group may enhance the antimicrobial activity of the polymer. Suitable polar pendant groups include, for example, N-hydroxymethylacrylamide, dimethylacrylamide, and alcohol groups.

In some embodiments, the antimicrobial polymer of the present disclosure does not comprise a pendant group that includes a carboxylate or alkoxylate chemical group.

Antimicrobial polymers of the present disclosure can be synthesized by reacting, in an organic solvent, monomers comprising the pendant groups. Suitable monomers for the reaction include, for example, acrylate monomers, methacrylate monomers, and combinations thereof. Other suitable monomers for the reaction include vinyl monomers and olefinic monomers.

The monomers can be combined, on a weight percent basis, in various ratios in the reaction. In some embodiments, the monomer comprising the quaternary ammonium pendant group can comprise from about 20% to about 80% of the monomers reacted to form a polymer. In some embodiments, the monomer comprising the quaternary ammonium pendant group can comprise greater than 20% of the monomers reacted to form a polymer. In some embodiments, the monomer comprising the quaternary ammonium pendant group can comprise greater than 30% of the monomers reacted to form a polymer. In some embodiments, the monomer comprising the quaternary ammonium pendant group can comprise greater than 40% of the monomers reacted to form a polymer. In some embodiments, the monomer comprising the quaternary ammonium pendant group can comprise greater than 50% of the monomers reacted to form a polymer. In some embodiments, the monomer comprising the quaternary ammonium pendant group can comprise greater than 60% of the monomers reacted to form a polymer. In some embodiments, the monomer comprising the quaternary ammonium pendant group can comprise greater than 70% of the monomers reacted to form a polymer. In some embodiments, the monomer comprising the quaternary ammonium pendant group can comprise 70% to 80% of the monomers reacted to form a polymer.

In some embodiments, the monomer comprising the nonpolar pendant group can comprise from about 20% to about 60% of the monomers reacted to form a polymer. In some embodiments, the monomer comprising the quaternary ammonium pendant group can comprise greater than 20% of the monomers reacted to form a polymer. In some embodiments, the monomer comprising the quaternary ammonium pendant group can comprise greater than 30% of the monomers reacted to form a polymer. In some embodiments, the monomer comprising the quaternary ammonium pendant group can comprise 30% to 40% of the monomers reacted to form a polymer.

In some embodiments, the monomer comprising the organosilane pendant group can comprise from about 1% to about 20% of the monomers reacted to form a polymer. In some embodiments, the monomer comprising the quaternary ammonium pendant group can comprise greater than 2% of the monomers reacted to form a polymer. In some embodiments, the monomer comprising the quaternary ammonium pendant group can comprise greater than 5% of the monomers reacted to form a polymer. In some embodiments, the monomer comprising the quaternary ammonium pendant group can comprise greater than 10% of the monomers reacted to form a polymer. In some embodiments, the monomer comprising the quaternary ammonium pendant group can comprise greater than 15% of the monomers reacted to form a polymer. In some embodiments, the monomer comprising the quaternary ammonium pendant group can comprise 15% to 20% of the monomers reacted to form a polymer.

In some embodiments, the reaction mixture used to make the antimicrobial polymer comprises at least 20% monomers comprising a quaternary ammonium pendant group, at least 20% monomers comprising a nonpolar pendant group, and at least 2% monomers comprising an organosilane pendant group.

The monomers are mixed in an organic solvent and are reacted under conditions suitable to form a polymer. For example, the reaction mixture can be purged with nitrogen to remove other dissolved gasses. In some embodiments, the reaction mixture can be sealed, heated, and mixed (e.g., mixed at 65° C) for a period of time sufficient to allow polymerization of, for example, at least 99.5% of the monomers. In some embodiments, an additional initiator (e.g., 2,2-Azobis(2-methylbutyronitrile), available from DuPont of Wilmington, Delaware, USA, under the trade name Vazo-67) can be added to the mixture to react with any unreacted monomers from the original mixture. The extent of the reaction of monomers can be determined by, for example, a calculation of the percent solids in the mixture. The antimicrobial polymer typically comprises about 25 weight percent (wt%) of the solution in which it is made.

### Adhesion-promoting reagents:

In any embodiment the method of making an antimicrobial coating according the present disclosure, one or more adhesion-promoting reagent can be used in the process. Suitable adhesion-promoting reagents include organosilane compounds having a silane group that can react to form Si-O-Si linkages and a leaving group (e.g. an alkoxy group).

The adhesion-promoting reagent can form Si-O-Si linkages with another organosilane compound (e.g., an unreacted organosilane compound of the present disclosure), an organosilane-containing polymer (e.g., the antimicrobial polymers of the present disclosure), and/or a siliceous substrate (e.g., glass). Advantageously, the adhesion-promoting reagents promote improved adhesion of the antimicrobial coatings by increasing the number of attachment points (to the substrate) per antimicrobial molecule. Furthermore, the adhesion-promoting reagents promote improved durability of the antimicrobial coatings by increasing the number of intramolecular linkages per antimicrobial polymer molecule and/or the number of linkages between the antimicrobial polymer and the substrate.

In addition to promoting the formation of Si-O-Si bonds between the organosilane compounds in the coating compositions of the present disclosure, the preferred adhesion-promoting reagents can also be used as an adhesion promoter to increase the interfacial adhesion between the substrate and the antimicrobial polymer composition of the present disclosure.

Nonlimiting examples of suitable adhesion-promoting reagents include N-2(aminoethyl)-3-aminopropylmethyldimethoxysilane, N-2-(aminoethyl)-3-aminopropyltrimethoxysilane, N-2-(aminoethyl)-3-aminopropyltriethoxysilane, 3-aminopropyltrimethoxysilane, 3-aminopropyltriethoxysilane, 3-triethoxysilyl-N-(1,3-dimethyl-butyliden)propylamine, and N-phenyl-3-aminopropyltrimethoxysilane. In view of the present disclosure, other suitable adhesion-promoting reagents will be apparent to a person having ordinary skill in the art.

Other suitable adhesion-promoting reagents are disclosed in U.S. Patent Publication no. US 2008/0064825. For example, amino-substituted organosilane esters (e.g., alkoxy silanes) are preferred adhesion-promoting reagents. The antimicrobial articles of the present disclosure may be made by reacting an amino-substituted organosilane ester or ester equivalent and an antimicrobial polymer that has a plurality of polar functionalities combinatively reactive with the silane ester or ester equivalent. The amino-substituted organosilane ester or ester equivalent bears on the silicon atom at least one ester or ester equivalent group, preferably 2, or more preferably 3 groups. Ester equivalents are well known to those skilled in the art and include compounds such as silane amides (RNR'Si), silane alkanoates (RC(O)OSi), Si-0-Si, SiN(R)-Si, SiSR and RCONR'Si. These ester equivalents may also be cyclic such as those derived from ethylene glycol, ethanolamine, ethylenediamine and their amides. R and R' are defined as in the "ester equivalent" definition herein.

3-aminopropyl alkoxysilanes are well known to cyclize on heating and these RNHSi compounds would be useful in this invention. Preferably, the amino-substituted organosilane ester or ester equivalent has ester groups such as methoxy that are easily volatilized as methanol so as to avoid leaving residue at the interface which may interfere with bonding. The amino-substituted organosilane must have at least one ester equivalent; for example, it may be a trialkoxysilane.

For example, the amino-substituted organosilane may have the formula: ZNH-L-SiX'X"X"', where Z is hydrogen, alkyl, or substituted alkyl including amino-substituted alkyl; where L is a divalent straight chain C1-12 alkylene or may comprise a C3-8 cycloalkylene, 3-8 membered ring heterocycloalkylene, C2-12 alkenylene, C4-8 cycloalkenylene, 3-8 membered ring heterocycloalkenylene or heteroarylene unit. L may be interrupted by one or more divalent aromatic groups or heteroatomic groups. The aromatic group may include a heteroaromatic. The heteroatom is preferably nitrogen, sulfur or oxygen. L is optionally substituted with C1-4 alkyl, C2-4 alkenyl, C2-4 alkynyl, C1-4 alkoxy, amino, C3-6 cycloalkyl, 3-6 membered heterocycloalkyl, monocyclic aryl, 5-6 membered ring heteroaryl, C1-4 alkylcarbonyloxy, C1-4 alkyloxycarbonyl, C1-4 alkylcarbonyl, formyl, C1-4 alkylcarbonylamino, or C1-4 aminocarbonyl. L is further optionally interrupted by -O-, -S-, -N(Rc)-, -N(Rc)-C(O)-, -N(Rc)---C(O)-O-, -O-C(O)-N(Rc )-, -N(Rc )-C(O)-N(Rd)-, - O-C(O)-, -C(O)-O-, or -O-C(O)-O-. Each of Rc and Rd, independently, is hydrogen, alkyl, alkenyl, alkynyl, alkoxyallcyl, aminoalkyl (primary, secondary or tertiary), or haloalkyl; and each of X', X" and X'" is a C1-18 alkyl, halogen, C1-8 alkoxy, C1-8 alkylcarbonyloxy, or amino group, with the proviso that at least one of X', X", and X'" is a labile group. Further, any two or all of X', X" and X'" may be joined through a covalent bond. The amino group may be an alkylamino group. Examples of amino-substituted organosilanes include 3-triethoxysilyl-N-(1,3-dimethyl-butyliden)propylamine, N-phenyl-3-aminopropyltrimethoxysilane, and 3-[2-(2-aminoethylamino)ethylamino]propyltrimethoxysilane, 3-aminopropyltrimethoxysilane (SILQUEST A-1110), 3-aminopropyltriethoxysilane (SILQUEST A-1100), 3-(2-aminoethyl)aminopropyltrimethoxysilane (SILQUEST A-1120), SILQUEST A-1130, (aminoethylaminomethyl)phenethyltrimethoxysilane, (aminoethylaminomethyl) phenethyltriethoxysilane, N-(2-aminoethyl)-3-aminopropylmethyldimethoxysilane (SILQ UEST A-2120), bis-(γ-triethoxysilylpropyl) amine (SILQUEST A-1170), N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3-aminopropylmethyldiethoxysilane, tetraethoxysilane and oligomers thereof, methyltriethoxysilane and oligomers thereof, oligomeric aminosilanes such as DYNASYLAN 1146, 3-(N-methylamino)propyltrimethoxysilane, N-(2-aminoethyl)-3-aminopropylmethyldimethoxysilane, N -(2-aminoethyl)-3-aminopropylmethyldiethoxysilane, N-(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.

Additional "precursor" compounds such as a bis-silyl urea [RO)₃Si(CH₂)NR]₂C=O are also examples of amino-substituted organosilane ester or ester equivalent that liberate amine by first dissociating thermally. The amount of aminosilane is between 0.01% and 10% by weight relative to the functional polymer, preferably between 0.03% and 3%, and more preferably between 0.1 % and 1%.

In some embodiments, the adhesion-promoting reagents can be added to a coating mixture comprising a first organosilane and a liquid crystal silane, as disclosed herein, and contacted with a substrate (e.g., a glass substrate) under conditions that facilitate the formation of Si-O-Si linkages, as described herein. The coating mixture can be contacted with a suitable substrate, as described herein. Accordingly, the silane group in the adhesion-promoting reagent can link a first organosilane molecule to another first organosilane molecule (which may optionally be a component of a polymeric structure), a quaternary ammonium organosilane (e.g., the liquid crystal silane disclosed in U.S. Patent No. 6,504,582) molecule (which may optionally be a component of a polymeric structure); or the substrate; or the silane group in the adhesion-promoting reagent can link a liquid crystal silane molecule to another liquid crystal silane molecule (which may optionally be a component of a polymeric structure) or to the substrate.

In some embodiments, the adhesion-promoting reagents can be added to a coating mixture comprising a polymer having a plurality of pendant groups that include a first pendant group that includes a first quaternary ammonium component, a second pendant group that includes a nonpolar component, and subsequently a third pendant group that includes an organosilane or organic silane ester component. Optionally, the coating mixture further can comprise a first organosilane, as described herein. The coating mixture can be contacted with a suitable substrate and heated as described herein to facilitate the formation of Si-O-Si bonds.

In an alternative embodiment, one or more adhesion-promoting reagent can be dissolved in an organic solvent and coated onto a suitable substrate (e.g., glass) as described herein to form a first coating. After removal of the solvent by evaporation, the substrate comprises a layer (i.e., a "primer layer" or "adhesion-promoting" layer) of the adhesion-promoting reagent coated thereon. Subsequently, a composition (e.g., a solution) comprising any antimicrobial polymer of the present disclosure in organic solvent can be coated onto the primer layer. After removal of the solvent by evaporation, the substrate now comprises two layers, the "primer layer" and the antimicrobial polymer layer. The substrate, now comprising two coated layers, can be heated (e.g., to about 120 degrees C for about 3 minutes to about 15 minutes) to facilitate the formation of Si-O-Si bonds and, thereby, covalently couple the polymer to the substrate.

### Catalysts:

In any embodiment the method of making an antimicrobial coating according the present disclosure, one or more catalyst can be used in the process. Suitable catalysts include any compound that promotes the formation of Si-O-Si bonds. Nonlimiting examples of suitable catalysts include an acid (e.g., an organic acid), a base (e.g., an organic base), tin octoate and 1,8-Diazabicycloundecene (DBU). In any embodiment, the catalyst can be added with the antimicrobial component and the adhesion-promoting reagent, if present, to the first composition described herein.

In use, the catalyst can be dissolved in the first composition, second composition, first mixture and/or second mixture described herein. Typically, the final concentration of the catalyst in any coating composition is relatively low (e.g., about 0.04 weight percent). A person of ordinary skill in the art will recognize that the concentration of the catalyst should be sufficiently high enough to catalyze the cross-linking reaction, while avoiding substantial interference with the optical properties (e.g., color) of the coating and/or interference with the shelf-life of the coating mixture.

### Substrates and Articles:

Antimicrobial polymers of the present disclosure can be applied as a coating to a variety of substrates. Useful substrates include, for example, non-siliceous ceramic materials, siliceous materials such as glasses and siliceous ceramic materials, metals, metal oxides, natural and man-made stones, woven and non-woven fabrics, wood, and polymeric materials that are either thermoplastic polymers or thermoset polymers. Exemplary polymeric substrates include, but are not limited to, rayon polyester, polyethylene terephthalate (PET), poly(meth)acrylates, polycarbonates, polystyrenes, polystyrene copolymers such as styrene acrylonitrile copolymers, polyesters, polyethersulfone, acrylics and acrylic copolymers, polyacrylamides, and polyurethanes, and combinations thereof. Suitable natural polymer substrates include, for example, polylactic acid (PLA), polyglycolic acid (PGA), wood pulp, cotton, cellulose, rayon, and combinations thereof.

The substrates can be used to fabricate a variety of useful articles (e.g., as a part, a portion, or the entirety of the article). The articles comprise a variety of surfaces that may be deliberately or incidentally contacted with microbiologically-contaminated items during routine use. The articles include, for example, electronic displays (e.g., computer touch screens). Suitable articles may be found in food-processing environments (e.g., food-processing rooms, equipment, countertops) and health care environments (e.g., patient care rooms, countertops, bedrails, patient care equipment such as instruments and stethoscopes, and in-dwelling medical devices such as urinary catheters and endotracheal tubes).

### Methods of Preparing Antimicrobial Coated Articles:

The present disclosure provides methods for coating the antimicrobial polymer of the present disclosure onto a substrate. The composition (e.g., a reaction mixture in a solution) comprising the antimicrobial polymer in solvent (e.g., an aqueous solvent, an organic solvent) can be contacted with a substrate. The solvent can be evaporated to leave the antimicrobial polymer in the form of a coating on the substrate. In some embodiments, the substrate can be heated before and/or during the contacting step to accelerate the evaporation of the solvent. Preferably, the substrate is heated to a temperature that does not degrade the function of the polymer or a component of the subtrate onto which the polymer is coated. A suitable temperature for contacting the polymer solution on a glass subtrate is from room temperature to about 120°C. A person of ordinary skill in the art will recognize that higher temperatures will facilitate faster removal of organic solvent from the polymer solution.

In some embodiments, the antimicrobial polymer can be diluted to a final concentration of 1 wt.% to about 20 wt% in the organic solvent before using the diluted solution to coat the antimicrobial polymer onto a substrate. In some embodiments, the antimicrobial polymer is diluted to a final concentration of 1 wt% to about 5 wt% in the organic solvent before using the diluted solution to coat the antimicrobial polymer onto a substrate. Suitable organic solvents to dilute the polymer have a flashpoint below 150° C and include ethers, ketones esters and alcohols, for example, isopropyl alcohol.

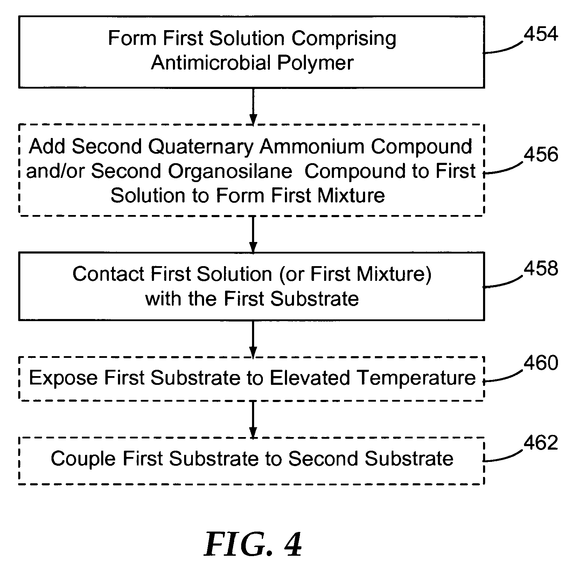

Turning back to the drawings, FIG. 4 shows one embodiment of a method of preparing a coated article according to the present disclosure. The method includes the step 454 of forming a first composition comprising an antimicrobial polymer in a solvent. The polymer can be formed by mixing a plurality of monomers in a suitable solvent (e.g., an organic solvent such as isopropyl alcohol, for example) as disclosed herein. Preferably, a relatively small portion (e.g., 3%) of the solvent comprises acidified water. Acidified water in the reaction mixture can facilitate bonding between silane groups. Optionally, after forming the antimicrobial polymer, the polymer composition can be diluted (not shown) in a solvent, as described above, before contacting it with a substrate.

The method may include optional step 456 of mixing the first composition with a second quaternary ammonium compound and/ or a second organosilane compound to form a first mixture. Suitable second quaternary ammonium compounds are described in U.S. Patent No. 6,504,583; and include antimicrobial silanated quaternary amine compounds such as N,N-dimethyl-N-(3-(trimethoxysilyl)propyl)-1-octadecanaminium chloride (CAS Number 27668-52-6), for example. Suitable second organosilane compounds comprise hydrolyzable groups and can facilitate the formation of crosslinks between silanated compounds and/or crosslinks between silanated compounds and a siliceous substrate. Examples of suitable second organosilane compounds include alkyl halide organosilane compounds and trimethoxysilyl compounds (e.g., 3-chloropropyltrimethoxysilane).

The method further comprises the step 458 of contacting the first composition with the first substrate under conditions suitable to permit bonding between the antimicrobial polymer and the first substrate. Initially, the frst composition, which may optionally include an antimicrobial monomer, is applied to a first substrate. The first substrate may be any of the suitable substrates disclosed herein. In some embodiments, the first substrate may be a coating (e.g., a siliceous coating) on a second substrate (e.g., a polymer or glass substrate). In some embodiments, the first substrate may be glass, a polymer film, or a diamond-like glass material. Suitable diamond-like glass materials are described in U.S. Patent Nos. 6,696,157; 6,015,597; and 6,795,636; and U.S. Patent Publication No. US 2008/196664. The first composition may be applied by a variety of processes known in the art such as, for example, wiping, brushing, dip coating, curtain coating, gravure coating, kiss coating, spin coating, and spraying.

Contacting the first composition with the substrate further comprises contacting the first composition under conditions that facilitate the formation of Si-O-Si bonds. A person having ordinary skill in the art will appreciate that during and after the period which solvent of the first composition evaporates, components of the first composition will begin reacting with each other and/or with the siliceous substrate to form Si-O-Si bonds. This reaction will proceed relatively slowly at ambient temperature (circa 23° C). Heating the substrate can facilitate the formation of cross-linking covalent bonds between the silane groups in the antimicrobial coating composition and the silane groups on the surface of the first substrate. Thus, in certain preferred embodiments, the formation of the Si-O-Si bonds can be accelerated by the optional step 460 of exposing the coated substrate to an elevated temperature. Without being bound by theory, other forces (e.g., hydrophobic interaction, electrostatic forces, hydrogen bonding, and/or adhesion) also may facilitate coupling of components of the antimicrobial coating composition to the first substrate.

In general, exposing the first substrate to higher temperatures while contacting it with the polymer composition will require shorter times for the solvent to evaporate and for the polymer to bond to the first substrate. However, the contacting step should be performed at temperatures below which the siloxane bonds dissociate. For example, in some embodiments, the contacting step can be conducted at about ambient temperature (20-25 °C) for about 10 minutes to about 24 hours. In some embodiments, the contacting step can be conducted at about 130 °C for about 30 seconds to about 3 minutes. The conditions for the contacting step can have a significant impact on the properties of the polymer coating on the substrate. For example, a polymer contacted ("cured") at room temperature for 24 hours can be measurably more hydrophobic than a polymer cured at about 130 °C for about 3 minutes. In some embodiments, the hydrophobicity of the coating correlates with the durability of the polymer coating on the substrate.

In some embodiments (not shown), the method optionally includes pre-treatments of the substrates by priming, plasma etching, corona for interfacial adhesion of the coating to substrates.

In some embodiments (not shown), the method optionally includes post treatments of the coating by heating or irradiaitons including UV, IR plasma, E-beam for further improvent of interfacial adhesion of the coating to substrates. These treatments can promote inter-polymer crosslinking, as well as increase the number of covalent linkages between the polymer and the substrate, thereby improving the durabililty of the coating on the surface of the substrate. If the first substrate is exposed to an elevated temperature (step 460), the method may include cooling the substrate. Typically, the substrate is cooled to room temperature.

In some embodiments, the method optionally includes the step 462 of coupling the first substrate to a second substrate. The first substrate may be coupled to the second substrate before or after the step 458 of contacting the first composition or first mixture with the first substrate. The second substrate may be any suitable substrate described herein. For example, the second substate may be a glass layer or particle and the first substrate may be a glass or diamond-like coating and the polymer may be applied to the first substrate after the first substrate is coated onto the second substrate. In an alternative embodiment, the first substrate may be a polymer film with adhesive on one major surface of the film. In the alternative embodiment, the polymer composition may be applied to the major surface of the film opposite the adhesive and the polymer-coated adhesive film may subsequently be coupled via the adhesive to a second substrate such as a glass or polymer layer, for example.

In some embodiments, the method further comprises a step of applying a siliceous layer to the first substrate. The first substrate may be any suitable substrate described herein to which a siliceous layer may be applied. The siliceous layer may be applied by methods that are known to a person of ordinary skill in the art. A nonlimiting example of applying a siliceous layer to a substrate is described in Example 1 of U.S. Patent No. 7,294,405; wherein an antiglare hard coat siliceous layer is applied to a glass substrate. In these embodiments, the method further includes forming a first composition comprising the antimicrobial polymer in a solvent, as described above, for example. Optionally, the these embodiments may include the step of mixing the first composition with asecond quaternary ammonium compound to form a first mixture, as described above, for example. The method further includes contacting the first composition or first mixture to the siliceous layer. The first composition or first mixturecan be applied by any suitable coating method, such as the coating methods described herein, for example. Contacting the first composition with the siliceous layer further comprises contacting the first composition with the siliceous layer under conditions suitable to facilitate the formation of Si-O-Si bonds, as described herein. Optionally, contacting the first composition with the first substrate may further comprise treating the polymer-coated substrate with actinic and/or ionizing radiation (e.g., ultraviolet light, e-beam, plasma, or the like). This treatment can promote inter-polymer crosslinking, as well as increase the number of covalent linkages between the polymer and the substrate, thereby improving the durabililty of the coating on the surface of the substrate.

It should be noted that, in any embodiment of the methods disclosed herein, pretreatment of siliceous layers or substrates prior to applying antimicrobial polymer compositions of the present disclosure can improve the bonding between the polymer and the substrate (e.g., siliceous material). Pretreatment of the siliceous layer or the substrate may include, for example, soaking the layer or the substrate in a volatile solvent (e.g., water, isopropyl alcohol) and/or wiping the layer or the substrate with the volatile solvent. Optionally, the solvent may further comprise a solution of a basic compound such as potassium hydroxide, for example. In some embodiments, the solvent may be saturated with the solution of the basic compound.

In particular, pretreatments that include heating the siliceous layer or the substrate from about 100 degrees to about 150 degrees for 20 minutes to 60 minutes can improve the bonding between the polymer and the substrate. Other suitable heat treatments include exposing the siliceous substrate to a temperature from about 475 to about 550 degrees C for a duration of at least about 3 minutes or longer; preferably, for about 3 minutes to about 10 minutes; more preferably, for about 6 minutes to about 10 minutes. In some embodiments, pretreatment by heating the substrate shortly before the antimicrobial coating is applied results in improved bonding (e.g., as measured by the durability of the coating) between the coating and the substrate. The improved bonding can result in significantly greater durability of the polymer layer on the substrate. This can be demonstrated, for example, using the Eraser Test described herein. Without being bound by theory, it is believed that pretreatment of the substrate by heating removes excess moisture and other impurities (e.g., organic residues) present on the surface of the substrate (e.g., siliceous material) and provides greater ability of the surface silane groups to react with the silanated polymers and/or compounds in the coating compositions disclosed herein.

### EMBODIMENTS

Embodiment A is an article, comprising:
   an organic polymer having a plurality of pendant groups comprising
      a first pendant group comprising a first quaternary ammonium component;
      a second pendant group comprising a nonpolar component;
      a third pendant group comprising a first organosilane component; and
   a touch-sensitive substrate comprising a surface;
   wherein the organic polymer is coupled to the surface.
Embodiment B is the article of embodiment A, further comprising a siliceous substrate that includes a first side and a second side, wherein the organic polymer is coupled to the first side of the siliceous substrate, and wherein the touch-sensitive substrate is coupled to the second side of the siliceous substrate.
Embodiment C is the article of embodiment A or embodiment B,wherein the article does not comprise a conductive layer.
Embodiment D is the article of any one of the preceding embodiments, wherein the organic polymer further comprises a second quaternary ammonium component.
Embodiment E is the article of any one of the preceding embodiments, wherein the organic polymer further comprises a second organosilane component.
Embodiment F is the article of embodiment E, wherein the second organosilane component comprises an alkyl halide.
Embodiment G is the article of any one of the preceding embodiments wherein, in the organic polymer, the ratio of the number of N atoms associated with the first quaternary ammonium component and second quaternary ammonium component, if present, and the number of Si atoms associated with the first organosilane component and second organosilane component, if present, is about 0.1:1 to about 10:1.
Embodiment H is the article of any one of the preceding embodiments, wherein at least one of the pendant components comprises a fluorochemical.
Embodiment I is the article of embodiment H, wherein the second pendant group comprises a fluorochemical.
Embodiment J is the article of any one of the preceding embodiments, wherein the contact angle of deionized water deposited on the cured polymer is about 80° to about 120°, using the ASTM D 7334.7606-1 test method.
Embodiment K is the article of embodiment J, wherein the contact angle of deionized water deposited on the cured polymer is about 85° to about 110°, as measured by ASTM test method number D 7334.7606-1.
Embodiment L is the article of any one of the preceding embodiments, wherein the scratch resistance of the cured polymer is about #5 to about #8 Moh's hardness, as measured by ASTM test method number D 7027.26676.
Embodiment M is the article of any one of the preceding embodiments, wherein the surface to which the organic polymer is coupled is a glass or a polymeric surface.
Embodiment N is the article of any one of the preceding embodiments, wherein the siliceous substrate is covalently coupled to the touch-sensitive substrate.
Embodiment O is the article of any one of embodiments A through P, wherein the substrate further comprises an antiglare component.
Embodiment P is a method of making a coated article, the method comprising:
   forming a first composition of an organic polymer in organic solvent, the polymer having a plurality of pendant groups comprising,
      a first pendant group comprising a first quaternary ammonium component,
      a second pendant group comprising a nonpolar component, and
      a third pendant group comprising a first organosilane component;
   mixing a second quaternary ammonium component with the first composition to form a first mixture; and
   contacting the first mixture with a substrate under conditions suitable to form covalent linkages between the organic polymer, the substrate, and the second quaternary ammonium component.
Embodiment Q is the method of embodiment P, wherein forming a first mixture further comprises forming a first mixture that includes a catalyst compound.
Embodiment R is the method of embodiment P or embodiment Q, wherein forming a first mixture further comprises forming a first mixture comprising a second organosilane component.
Embodiment S is the method of any one of embodiments P through R, further comprising coupling the substrate to a touch-sensitive substrate.
Embodiment T is the method of embodiment S, wherein coupling the siliceous substrate to the touch-sensitive substrate comprises covalently coupling the siliceous substrate to the touch-sensitive substrate.
Embodiment U is a method of making a coated article, the method comprising:
   forming a first composition of an organic polymer in organic solvent, the polymer having a plurality of pendant groups comprising,
      a first pendant group comprising a first quaternary ammonium component,
      a second pendant group comprising a nonpolar component, and
      a third pendant group comprising a first organosilane component;
   mixing an adhesion-promoting reagent with the first composition to form a second mixture; and
   contacting the second mixture with a substrate under conditions suitable to form covalent linkages between the organic polymer and the substrate.
Embodiment V is the method of embodiment U, wherein forming a second mixture further comprises forming a first composition that includes a catalyst compound.
Embodiment W is the method of embodiment U or embodiment V, wherein forming a second mixture further comprises forming a second mixture comprising a second quaternary ammonium component.
Embodiment X is the method of any one of embodiments U through W, wherein forming a second mixture further comprises forming a second mixture comprising a second organosilane component.
Embodiment Y is the method of any one of embodiments U through X, wherein the adhesion promoting reagent is selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl), phenethyltriethoxysilane, N-(2-aminoethyl)-3-aminopropylmethyldimethoxysilane, bis-(γ-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysi lane, 3-aminopropyltris(methoxyethoxyethoxy)silane, 3 -aminopropylmethyldiethoxysilane, tetraethoxysilane and oligomers thereof, methyltriethoxysilane and oligomers thereof; an oligomeric aminosilane, 6,3-(N-methylamino)propyltrimethoxysilane, N-(2-aminoethyl)-3-aminopropylmethyldimethoxysilane, N -(2-aminoethyl)-3-aminopropylmethyldiethoxysilane, N-(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.
Embodiment Z is The method of any one of embodiments P through Y, further comprising:
   contacting a second composition comprising an adhesion-promoting reagent in organic solvent with the siliceous substrate under conditions suitable to form covalent linkages between the adhesion-promoting reagent and the siliceous substrate;
   wherein contacting the second composition with the siliceous substrate occurs prior to contacting the first or second mixture with the siliceous substrate.
Embodiment AA is the method of embodiment Z, wherein contacting the second composition with the siliceous substrate further comprises contacting the second composition with the siliceous substrate at a temperature higher than 25° C.
Embodiment BB is a method of making a coated article, the method comprising:
   forming a first composition of an organic polymer in organic solvent, the polymer having a plurality of pendant groups comprising,
      a first pendant group comprising a first quaternary ammonium component,
      a second pendant group comprising a nonpolar component, and
      a third pendant group comprising a first organosilane component;
   mixing a second quaternary ammonium component with the first composition to form a first mixture; and
   contacting the first mixture with a touch-sensitive substrate under conditions suitable to form covalent linkages between organic polymer and the touch-sensitive substrate.
Embodiment CC is the method of embodiment BB, wherein forming a first mixture further comprises forming a first mixture that includes a catalyst compound.
Embodiment DD is the method of embodiment BB or embodiment CC, wherein mixing a second quaternary ammonium component with the first composition further comprises mixing a second organosilane component with the first composition.
Embodiment EE is the method of any one of embodiments BB through DD, further comprising, after the contacting step, rinsing the coated article.
Embodiment FF is a method of making a coated article, the method comprising:
   forming a first composition of an organic polymer in organic solvent, the polymer having a plurality of pendant groups comprising,
      a first pendant group comprising a first quaternary ammonium component,
      a second pendant group comprising a nonpolar component, and
      a third pendant group comprising a first organosilane component;
   mixing an adhesion-promoting reagent with the first composition to form a second mixture; and
   contacting the second mixture with a touch-sensitive substrate under conditions suitable to form covalent linkages between organic polymer, the touch-sensitive substrate, and the second quaternary ammonium component.
Embodiment GG is the method of embodiment FF, wherein forming a second mixture further comprises mixing a second quaternary ammonium component with the adhesion-promoting reagent and the first composition.
Embodiment HH is the method of embodiment FF or embodiment GG, wherein the adhesion-promoting reagent is selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(y-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.
Embodiment II is the method of any one of embodiments BB through HH, further comprising:
   providing a second composition comprising a adhesion-promoting reagent in organic solvent;
   contacting the second composition with the touch-sensitive substrate;
   wherein contacting a second composition with the touch-sensitive substrate occurs prior to contacting the first or second mixture with the siliceous substrate.
Embodiment JJ is an antimicrobial composition, comprising:
   an organic polymer having a plurality of pendant groups comprising
      a first pendant group comprising a first quaternary ammonium component;
      optionally, a second pendant group comprising a perfluorinated nonpolar component; and
      a third pendant group comprising a first organosilane component;
   with the proviso that the polymer does not comprise a pendant group that includes a carboxylate or alkoxylate chemical group.
Embodiment KK is the antimicrobial composition of embodiment JJ, further comprising a fourth pendant component, wherein the fourth pendant component comprises a polar chemical group.
Embodiment LL is a composition, comprising:
   a solvent;
   a polymer having a plurality of pendant groups comprising,
      a first pendant group comprising a first quaternary ammonium component,
      a second pendant group comprising a nonpolar component, and
      a third pendant group comprising a first organosilane component; and
   an adhesion-promoting reagent.
Embodiment MM is the composition of embodiment LL, wherein the adhesion-promoting reagent is selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(y-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.
Embodiment NN is an article, comprising:
   a touch-sensitive substrate comprising a surface;
   a first layer coated on the surface, the first layer comprising an adhesion-promoting reagent; and
   a second layer coated on the first layer, the second layer comprising an organic polymer having a plurality of pendant groups comprising
      a first pendant group comprising a first quaternary ammonium component;
      a second pendant group comprising a nonpolar component;
      a third pendant group comprising a first organosilane component.
Embodiment OO is the article of embodiment NN, wherein the adhesion-promoting reagent is selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(y-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N-(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.

The invention will be further illustrated by reference to the following non-limiting Examples. All parts and percentages are expressed as parts by weight unless otherwise indicated.

### EXAMPLES

The present invention is more particularly described in the following examples that are intended as illustrations only, since numerous modifications and variations within the scope of the present invention will be apparent to those skilled in the art. Unless otherwise noted, all parts, percentages, and ratios reported in the following examples are on a weight basis, and all reagents used in the examples were obtained, or are available, from the chemical suppliers described below, or may be synthesized by conventional techniques.

A list of reagents used in the following examples in shown in Table 1.

**Table 1.**

| **Abbreviation** | **Chemical Name** | **Source** |
|---|---|---|
| 2EHA | 2-Ethylhexyl acrylate | Dow; Midland, MI |
| A-174 | Methacryloylpropyl trimethoxy silane | Aldrich; Milwaukee, WI |
| AA | Acrylic acid | BASF; Florham, NJ |
| A1120 | N(beta-aminoethyl) gamma-aminopropyltrimethoxysilane | ShinEtsu; Akron, OH |
| AEM5700 | 3-(trimethoxysilyl)-propyldimethyloctadecyl ammonium chloride | Aegis Environmental, Midland, MI |
| BHT | 2,6-Di-tert-4-methyl phenol | Aldrich; Milwaukee, WI |
| C₁₆H₃₃Br | 1-bromohexyl decane | Chemtura Corporation, Bay Minette, AL |
| C4FA | Perfluorobutyl sulfonamide n-methyl ethyl acrylate | Made as described in US6,852,781 |
| DMAc | Dimethyl acrvlamide | Jarchem Industries, Inc. Newark, NJ |
| DMAEA | Dimethylaminoethyl acrylate | CIBA; Marietta, GA |
| DMAEA-C16Br | Dimethylaminoethyl acrylate C16 bromide | See Example 2 |
| DMAEA-MC1 | Dimethylaminoethyl acrylate methyl chloride | CIBA; Marietta, GA |
| DMAEMA | Dimethylaminoethyl methacrylate | CIBA; Marietta, GA |
| DMAEMA-C16Br | Dimethylaminoethyl methacrylate C16 bromide | See Example 1 |
| DMAEMA-MC1 | Dimethylaminoethyl methacrylate methyl chloride | CIBA; Marietta, GA |
| EOA | Methoxy polyethylene glycol acrylate | Shin Nakamura Chemicals; Wakayama, JP |
| EOMA | polyethyleneglycol monomethacrylate | Nippon Nyukazai Co; Tokyo, JP |
| EtOAc | Ethyl acetate | J.T. Baker; Austin, TX |
| EtOH | Ethanol | J.T. Baker; Austin, TX |
| HEMA | Hydoxyethyl methacrylate | Cyro Industries; Parsippany, NJ |
| HFPOA | Hexafluoropropylene oxide oligomer amidol acrylate | U.S. Patent Application Publication No. 2004/0077775 |
| HFPOMA | Hexafluoropropylene oxide oligomer amidol methacrylate | U.S. Patent Application Publication No. 2004/0077775 |
| IBMA | Isobutyl methacrylate | Lucite International, Inc. Cordova, TN |
| IOA | Iso-octyl acrylate | Sartomer USA, LLC; Exton, PA |
| IPA | Isopropyl alcohol | VWR; Houston, TX |
| MEHQ | 4-Methoxyphenol | Alfa Aesar, Ward Hill, MA |
| NHMAc | N-(hydroxymethyl)-acrylamide | Aldrich; Milwaukee, WI |
| NVP | N-Vinylpyrrolidinone | ISP Chemicals, Inc.;Calvarv City, KY |
| SnOA | Tin-octoate | Alfa Aesar, Ward Hill, MA |
| Vazo-67 | 2,2-Azobis(2-methylbutyronitrile) | Dupont; Wilmington, DE |

### EXAMPLE 1.

### Synthesis of DMAEMA-C₁₆Br monomer

In a clean reactor; fitted with an overhead condenser, mechanical stirrer, and a temperature probe; were charged 918 parts by weight of acetone, 807 parts of C₁₆H₃₃Br,) 415.5 parts of DMAEMA, 2.0 parts of BHT and 2.0 parts of MEHQ. The batch was stirred at 150 rpm and a mixed gas (90/10 O₂/N₂) was purged through the solution throughout the reaction scheme. The mixture was heated to 74° C for 18 hours. A sample was taken out for analysis by gas chromatography (GC) and which revealed the conversion of >98% of the reactants to the desired product. At this point 918 parts of EtOAc was added slowly with stirring at very high speed. A white solid started to precipitate out. The heating was stopped and the mixture was cooled to room temperature. The reaction precipitate was recovered by filtration and the white solid material was washed with 200 parts of cold EtOAc. The solid material was dried in a vacuum oven at 40°C for 8 hours. The dried product was analyzed by nuclear magnetic resonance (NMR) spectroscopy, which revealed the presence of>99.9% pure DMAEMA-C₁₆Br monomer.

### EXAMPLE 2.

### Synthesis of DMAEA-C₁₆Br monomer

In a clean reactor; fitted with an overhead condenser, mechanical stirrer, and a temperature probe; were charged 546 parts of acetone, 488 parts of C₁₆H₃₃Br, 225 parts of DMAEA, 1.0 parts of BHT and 1.0 parts of MEHQ. The batch was stirred at 150 rpm and a mixed gas (90/10 O₂/N₂) was purged through the solution throughout the reaction scheme. The mixture was heated to 74°C for 18 hours. A sample was taken out for analysis by GC and it revealed the conversion of >98% of the reactants to the desired product. At this point the reaction mixture heating was stopped and 1,000 parts of EtOAc was added slowly with stirring at very high speed. A white solid started to precipitate out. The mixture was allowed to cool to room temperature. The precipitate accumulated in the solution upon standing for couple of hours at room temperature. The reaction mixture was filtered and the white solid filtrate was washed with 1,000 parts of cold EtOAc. The white solid filtrate was dried in a vacuum oven at 40°C for 8 hours. The solid material was analyzed by NMR spectroscopy, which revealed the presence of>99.9% pure DMAEA-C₁₆Br monomer.

### EXAMPLES 3-39

### Synthesis of antimicrobial polymers

In a clean reaction bottle, the monomers (e.g., in Example 6, 50 parts of DMAEMA-C₁₆Br monomer, 10 parts of A-174 monomer, and 40 parts of IOA monomer) were combined with 0.5 parts of Vazo-67 and 300 parts of IPA. The mixture was purged with dry nitrogen for 3 minutes. The reaction bottle was sealed and placed in a 65°C preheated water bath with mixing. The reaction mixture was heated for 17 hours at 65°C with mixing. The viscous reaction mixture was analyzed for % solids. To drive the reaction of the residual monomer to >99.5% completion, an additional 0.1 parts of Vazo-67 was added to the mixture, the solution was purged and sealed. The bottle was placed in the 65°C water bath with mixing and heated for 8 hours. A conversion of (>99.5%) of the monomers was achieved, as evident by % solids calculation.

The polymers shown in Table 2 each were made according to this process. Table 2 lists the polymers that were synthesized in each Example. Comparative examples, which do not comprise an organosilane pendant group, are so designated in Table 2.

**Table 2 Antimicrobial polymers. The polymer designation (e.g., "p(DMAEMA-C16Br/A-174/IBMA)" in Example 4) refers to the combination of monomers used in the reaction mixture. Comparative examples, which do not comprise an organosilane pendant group, are designated with the notation "comparative".**

| **Example #** | **Polymer Designation** | **Monomer Ratio** |
|---|---|---|
| 3 comparative | p(DMAEMA-C₁₆Br/AA/IOA) | 50/20/30 |
| 4 | p(DMAEMA-C 16Br/A-174/IBMA) | 50/10/40 |
| 5 | p(DMAEA-MC1/A-174/IBMA) | 50/10/40 |
| 6 | p(DMAEMA-C16Br/A-174/IOA) | 50/10/40 |
| 7 comparative | p(DMAEMA-C₁₆Br/AA/IOA) | 50/20/30 |
| 8 | p(DMAEMA-C16Br/A-174/NHMAc/IOA | 50/10/10/30 |
| 9 comparative | p(DMAEMA-C16Br/HEMA/NHMAc/IOA | 50/10/10/30 |
| 10 | p(DMAEMA-C16Br/A-174/DMAc/IOA | 50/10/10/30 |
| 11 comparative | p(DMAEMA-C16Br/HEMA/DMAc/IOA | 50/10/10/30 |
| 12 comparative | p(DMAEMA-C16Br/AA/IBMA | 50/20/30 |
| 13 comparative | p(DMAEMA-C16Br/AA/2EHA | 50/20/30 |
| 14 comparative | p(DMAEMA-C 16Br/HFPOMA/AA) | 50/20/30 |
| 15 comparative | p(DMAEMA-C 16Br/HFPOMA/EOMA/AA) | 50/20/10/20 |
| 16 comparative | p(DMAEMA-C 16Br/HFPOMA/HEMA/AA) | 50/20/10/20 |
| 17 comparative | p(DMAEMA-C 16Br/HFPOMA/DMAc/AA) | 50/20/10/20 |
| 18 comparative | p(DMAEMA-C16Br/HFPOA/AA) | 50/20/30 |
| 19 comparative | p(DMAEMA-C16Br/HFPOA/HEMA/AA) | 50/20/10/20 |
| 20 comparative | p(DMAEMA-C 16Br/HFPOA/DMAc/AA) | 50/20/10/20 |
| 21 comparative | p(DMAEMA-C 16Br/HFPOA/EOA/AA) | 50/20/10/20 |
| 22 comparative | p(DMAEMA-C 16Br/HFPOA/IOA/AA) | 50/20/20/10 |
| 23 | p(DMAEA-C16Br/A-174/AA/IBMA) | 50/5/5/40 |
| 24 | p(DMAEA-C 16-Br/A-174/AA/IOA) | 50/5/5/40 |
| 25 | p(DMAEMA-C16Br/EOMA/A-174/HFPOMA) | 50/20/10/20 |
| 26 | p(DMAEMA-C16Br/HEMA/ A-174/HFPOMA) | 50/20/10/20 |
| 27 | p(DMAEMA-C16Br/A-174/NHMAc/IOA) | 50/10/10/30 |
| 28 | p(DMAEMA-C16Br/A-174/NVP/IOA) | 50/5/15/30 |
| 29 | p(DMAEMA-C16Br/A-174/NHMAc/IOA/EOA) | 50/5/10/10/25 |
| 30 | p(DMAEA-C16Br/A-174/NHMAc/IOA) | 50/10/10/30 |
| 31 | p(DMAEA-MC1/A-174/EOA/IOA) | 50/5/20/25 |
| 32 | p(DMAEMA-C16Br/A-174/IOA) | 50/10/40 |
| 33 comparative | p(DMAEMA-C 16Br/HFPOMA/HEMA/AA) | 50/20/10/20 |
| 34 | p(DMAEMA-C16Br/A-174/IOA/AA) | 50/10/30/10 |
| 35 | p(DMAEMA-C16Br/C4FA/A-174/AA | 50/20/10/20 |
| 36 | p(DMAEA-C16Br/A-174/IOA) | 50/10/40 |
| 37 | p(DMAEA-C16Br/A-174/IOA/AA | 50/10/30/10 |
| 38 | p(DMAEA-C16Br/DMAEA-MC1/A-174/IOA | 25/25/10/40 |
| 39 | p(DMAEA-C16Br/A-174/NVP/IOA | 50/5/15/30 |

### EXAMPLES 40-62

### Method of coating antimicrobial polymers onto conductive surface capacitive touch (SCT) sensor glass substrates

Conductively-coated glass (part number 29617) was obtained from Pilkington North America, Inc. (Toledo, OH). A glare-resistant hardcoat was applied to the glass according to the method described in Example 1 of U.S. Patent No. 7,294,405. The coated glass was cut into coupons, approximately 4" by 4" (10.2 cm by 10.2 cm), for coating and testing purposes.

Polymer solutions from the Examples shown in Table 3 were diluted in isopropyl alcohol to 5 wt% polymer. Approximately 5 milliliters of the diluted polymer solution were applied to a wipe (Sealed Edge Wiper 6259HC; Coventry, Kennesaw, GA), which was used immediately to manually distribute the polymer solution evenly over the surface of the glass coupon. The solvent was removed by heating the sample at 120 °C in a convection oven for 3-4minutes. After completely removing the solvent, the glass coupons were washed with soap (Optisolve OP7153-LF detergent; available from Kyzen North America (Manchester, NH) and deionized water in a 36" Billco Versa Clean Washer (Billco Manufacturing, Inc., Zelienople, PA) with attached fluid head and roller wash pan and dried. The dried samples were tested as described below.

**Table 3. Polymer-coated SCT glass substrates. Comparative examples, which do not comprise an organosilane pendant group, are designated with the notation "comparative".**

| Example No. | Polymer Designation | Polymer Example No. |
|---|---|---|
| 40 | p(DMAEMA-C16Br/A-174/IBMA) | 4 |
| 41 | p(DMAEA-MC1/A-174/IBMA) | 5 |
| 42 | p(DMAEMA-C16Br/A-174/IOA) | 6 |
| 43 comparative | p(DMAEMA-C 16Br/AA/IOA) | 7 |
| 44 | p(DMAEMA-C16Br/A-174/NHMAc/IOA) | 8 |
| 45 comparative | p(DMAEMA-C 16Br/HEMA/NHMAc/IOA) | 9 |
| 46 | p(DMAEMA-C16Br/A-174/DMAc/IOA) | 10 |
| 47 comparative | p(DMAEMA-C 16Br/HEMA/DMAc/IOA) | 11 |
| 48 comparative | p(DMAEMA-C 16Br/AA/IBMA) | 12 |
| 49 comparative | p(DMAEMA-C 16Br/AA/2EHA) | 13 |
| 50 comparative | p(DMAEMA-C 16Br/HFPOMA/AA) | 14 |
| 51 comparative | p(DMAEMA-C 16Br/HFPOMA/EOMA/AA) | 15 |
| 52 comparative | p(DMAEMA-C 16Br/HFPOMA/HEMA/AA) | 16 |
| 53 comparative | p(DMAEMA-C 16Br/HFPOMA/DMAc/AA) | 17 |
| 54 comparative | p(DMAEMA-C 16Br/HFPOA/AA) | 18 |
| 55 comparative | p(DMAEMA-C 16Br/HFPOA/HEMA/AA) | 19 |
| 56 comparative | p(DMAEMA-C 16Br/HFPOA/DMAc/AA) | 20 |
| 57 comparative | p(DMAEMA-C 16Br/HFPOA/EOA/AA) | 21 |
| 58 comparative | p(DMAEMA-C 16Br/HFPOA/IOA/AA) | 22 |
| 59 | p(DMAEA-C16BrA-174/AA/IBMA) | 23 |
| 60 | p(DMAEA-C16-Br/A-174/AA/IOA) | 24 |
| 61 | p(DMAEMA-C16Br/EOMA/A-174/HFPOMA) | 25 |
| 62 | p(DMAEMA-C16Br/HEMA/A-174/HFPOMA) | 26 |

### EXAMPLES 63-86

### Method of coating hybrid antimicrobial polymers onto conductive touch (SCT) glass substrates

AEM5700 antimicrobial solution was obtained from Aegis Environmental (Midland, MI). The AEM5700 was diluted to 1 wt% into IPA to make the working solution. Conductively-coated glass coupons were prepared as described in Examples 40-62. Polymer solutions from the Examples shown in Table 2 were diluted in IPA to 5 wt%. The diluted polymer solutions were mixed 1:1 with the working solution of AEM5700. The resulting mixtures (listed in Table 4) were applied to the glass coupons and the hybrid antimicrobial polymer-coated coupons were treated, cleaned, and dried as described in Examples 40-62.

A control (Example 86) consisted of applying 5 milliliters of the AEM5700 working solution directly to a glass coupon and treating, cleaning, and drying the coupon as described for Examples 59-62.

**Table 4. Hybrid antimicrobial polymer-coated SCT glass substrates. Comparative examples, which do not comprise an organosilane pendant group, are designated with the notation "comparative".**

| Example No. | Hybrid Polymer Designation | Polymer Example No. |
|---|---|---|
| 63 | p(DMAEMA-C 16Br/A-174/IBMA/AEM) | 4 |
| 64 | p(DMAEA-MC1/A-174/IBMA/AEM) | 5 |
| 65 | p(DMAEMA-C16Br/A-174/IOA/AEM) | 6 |
| 66 comparative | p(DMAEMA-C16Br/AA/IOA/AEM) | 7 |
| 67 | p(DMAEMA-C16Br/A-174/NHMAc/IOA/AEM) | 8 |
| 68 comparative | p(DMAEMA-C 16Br/HEMA/NHMAc/IOA/AEM) | 9 |
| 69 | p(DMAEMA-C16Br/A-174/DMAc/IOA/AEM) | 10 |
| 70 comparative | p(DMAEMA-C 16Br/HEMA/DMAc/IOA/AEM) | 11 |
| 71 comparative | p(DMAEMA-C 16Br/AA/IBMA/AEM) | 12 |
| 72 comparative | p(DMAEMA-C16Br/AA/2EHA/AEM) | 13 |
| 73 comparative | p(DMAEMA-C 16Br/HFPOMA/AA/AEM) | 14 |
| 74 comparative | p(DMAEMA-C16Br/HFPOMA/EOMA/AA/AEM) | 15 |
| 75 comparative | p(DMAEMA-C 16Br/HFPOMA/HEMA/AA/AEM) | 16 |
| 76 comparative | p(DMAEMA-C 16Br/HFPOMA/DMAc/AA/AEM) | 17 |
| 77 comparative | p(DMAEMA-C 16Br/HFPOA/AA/AEM) | 18 |
| 78 comparative | p(DMAEMA-C 16Br/HFPOA/HEMA/AA/AEM) | 19 |
| 79 comparative | p(DMAEMA-C 16Br/HFPOA/DMAc/AA/AEM) | 20 |
| 80 comparative | p(DMAEMA-C16Br/HFPOA/EOA/AA/AEM) | 21 |
| 81 comparative | p(DMAEMA-C16Br/HFPOA/IOA/AA/AEM) | 22 |
| 82 | p(DMAEA-C16BrA-174/AA/IBMA/AEM) | 23 |
| 83 | p(DMAEA-C 16-Br/A-174/AA/IOA/AEM) | 24 |
| 84 | p(DMAEMA-C16Br/EOMA/A-174/HFPOMA/AEM) | 25 |
| 85 | P(DMAEMA-C16Br/HEMA/ A-174/HFPOMA/AEM) | 26 |
| 86 | No Polymer - AEM5700 Control | - |

### EXAMPLES 87-109

### Method of coating antimicrobial polymers onto dispersive signal technology (DST) glass substrates

Chemstrengthened glass (2.2 mm flowed glass; part no. 37373.2) was obtained from EuropTec USA, Inc. (Clarksburg, WV). The glass was cut into coupons, approximately 4" by 4" (10.2 cm by 10.2 cm), for coating and testing purposes.

Polymer solutions from the Examples shown in Table 3 were diluted in isopropyl alcohol to 5 wt% polymer. Approximately 5 milliliters of the diluted polymer solution were applied to a wipe (Sealed Edge Wiper 6259HC; Coventry, Kennesaw, GA), which was used immediately to manually distribute the polymer solution evenly over the surface of the glass coupon. The solvent was removed by heating the sample at 120 °C in a convection oven for 3-4minutes. After completely removing the solvent, the glass coupons were washed with soap (Optisolve OP7153-LF detergent; available from Kyzen North America (Manchester, NH) and deionized water in a 36" Billco Versa Clean Washer (Billco Manufacturing, Inc., Zelienople, PA) with attached fluid head and roller wash pan and dried. The dried samples were tested as described below.

**Table 5. Polymer-coated DST glass substrates. Comparative examples, which do not comprise an organosilane pendant group, are designated with the notation "comparative".**

| Example No. | Polymer Designation | Polymer Example No. |
|---|---|---|
| 87 | p(DMAEMA-C16Br/A-174/IBMA) | 4 |
| 88 | p(DMAEA-MC1/A-174/IBMA) | 5 |
| 89 | p(DMAEMA-C 16Br/A-174/IOA) | 6 |
| 90 comparative | p(DMAEMA-C 16Br/AA/IOA) | 7 |
| 91 | P(DMAEMA-C16Br/A-174/NHMAc/IOA) | 8 |
| 92 comparative | P(DMAEMA-C 16Br/HEMA/NHMAc/IOA) | 9 |
| 93 | p(DMAEMA-C16Br/A-174/DMAc/IOA) | 10 |
| 94 comparative | p(DMAEMA-C 16Br/HEMA/DMAc/IOA) | 11 |
| 95 comparative | p(DMAEMA-C 16Br/AA/IBMA) | 12 |
| 96 comparative | p(DMAEMA-C 16Br/AA/2EHA) | 13 |
| 97 comparative | p(DMAEMA-C 16Br/HFPOMA/AA) | 14 |
| 98 comparative | P(DMAEMA-C 16Br/HFPOMA/EOMA/AA) | 15 |
| 99 comparative | P(DMAEMA-C 16Br/HFPOMA/HEMA/AA) | 16 |
| 100 comparative | P(DMAEMA-C 16Br/HFPOMA/DMAc/AA) | 17 |
| 101 comparative | p(DMAEMA-C 16Br/HFPOA/AA) | 18 |
| 102 comparative | p(DMAEMA-C 16Br/HFPOA/HEMA/AA) | 19 |
| 103 comparative | p(DMAEMA-C 16Br/HFPOA/DMAc/AA) | 20 |
| 104 comparative | p(DMAEMA-C 16Br/HFPOA/EOA/AA) | 21 |
| 105 comparative | p(DMAEMA-C 16Br/HFPOA/IOA/AA) | 22 |
| 106 | p(DMAEA-C16BrA-174/AA/IBMA) | 23 |
| 107 | p(DMAEA-C16-Br/A-174/AA/I OA) | 24 |
| 108 | p(DMAEMA-C 16Br/EOMA/A-174/HFPOMA) | 25 |
| 109 | p(DMAEMA-C16Br/HEMA/ A-174/HFPOMA) | 26 |

### EXAMPLES 110-133

### Method of coating hybrid antimicrobial polymers onto dispersive signal technology (DST) glass substrates

AEM5700 antimicrobial solution was obtained from Aegis Environmental (Midland, Michigan). The AEM5700 was diluted to 1 wt% into IPA to make the working solution. DST glass coupons were prepared as described in Examples 87-109. Polymer solutions from the Examples shown in Table 2 were diluted in IPA to 5 wt%. The diluted polymer solutions were mixed 1:1 with the working solution of AEM5700. The resulting mixtures (shown in Table 6) were applied to the glass coupons and the hybrid antimicrobial polymer-coated coupons were treated, cleaned, and dried as described in Examples 87-109 and were tested as described below.

A control (Example 133) consisted of applying 5 milliliters of the AEM5700 working solution directly to a glass coupon and treating, cleaning, and drying the coupon as described for Examples 87-109.

**Table 6. Hybrid antimicrobial polymer-coated DST glass substrates. Comparative examples, which do not comprise an organosilane pendant group, are designated with the notation "comparative".**

| Example No. | Hybrid Polymer Designation | Polymer Example No. |
|---|---|---|
| 110 | p(DMAEMA-C16Br/A-174/IBMA/AEM) | 4 |
| 111 | p(DMAEA-MC1/A-174/IBMA/AEM) | 5 |
| 112 | p(DMAEMA-C16Br/A-174/IOA/AEM) | 6 |
| 113 comparative | p(DMAEMA-C16Br/AA/IOA/AEM) | 7 |
| 114 | p(DMAEMA-C16Br/A-174/NHMAc/IOA/AEM) | 8 |
| 115 comparative | p(DMAEMA-C 16Br/HEMA/NHMAc/IOA/AEM) | 9 |
| 116 | p(DMAEMA-C16Br/A-174/DMAc/IOA/AEM) | 10 |
| 117 comparative | p(DMAEMA-C 16Br/HEMA/DMAc/IOA/AEM) | 11 |
| 118 comparative | p(DMAEMA-C16Br/AA/IBMA/AEM) | 12 |
| 119 comparative | p(DMAEMA-C16Br/AA/2EHA/AEM) | 13 |
| 120 comparative | p(DMAEMA-C 16Br/HFPOMA/AA/AEM) | 14 |
| 121 comparative | p(DMAEMA-C 16Br/HFPOMA/EOMA/AA/AEM) | 15 |
| 122 comparative | p(DMAEMA-C16Br/HFPOMA/HEMA/AA/AEM) | 16 |
| 123 comparative | p(DMAEMA-C16Br/HFPOMA/DMAc/AA/AEM) | 17 |
| 124 comparative | p(DMAEMA-C16Br/HFPOA/AA/AEM) | 18 |
| 125 comparative | p(DMAEMA-C16Br/HFPOA/HEMA/AA/AEM) | 19 |
| 126 comparative | p(DMAEMA-C 16Br/HFPOA/DMAc/AA/AEM) | 20 |
| 127 comparative | p(DMAEMA-C 16Br/HFPOA/EOA/AA/AEM) | 21 |
| 128 comparative | p(DMAEMA-C 16Br/HFPOA/IOA/AA/AEM) | 22 |
| 129 | p(DMAEA-C16BrA-174/AA/IBMA/AEM) | 23 |
| 130 | p(DMAEA-C16-Br/A-174/AA/IOA/AEM) | 24 |
| 131 | p(DMAEMA-C 16Br/EOMA/A-174/HFPOMA/AEM) | 25 |
| 132 | p(DMAEMA-C16Br/HEMA/ A-174/HFPOMA/AEM) | 26 |
| 133 | No Polymer - AEM5700 Control | - |

### EXAMPLES 134-143

### Method of coating antimicrobial polymers onto projected capacitive touch (PCT) sensor glass substrates

TPK antiglare top glass (1.1 mm thick; part no. AG-90) was obtained from TPK Touch Solutions (Xiamen Headquarters, Fujian, China)). The glass was cut into coupons, approximately 4" by 4" (10.2 cm by 10.2 cm), for coating and testing purposes. Just prior to coating with the antimicrobial polymer solutions, the glass coupons were heated to 130 °C in a convection oven for 30 minutes.

Polymer solutions from the Examples shown in Table 3 were diluted in isopropyl alcohol to 5 wt% polymer. Approximately 5 milliliters of the diluted polymer solution were applied to a wipe (Sealed Edge Wiper 6259HC; Coventry, Kennesaw, GA), which was used immediately to manually distribute the polymer solution evenly over the surface of the PCT glass coupon. In Examples 134-138, the solvent was removed by allowing the samples to air-dry at 20 ° C for 24 hours. In Examples 139-143, the solvent was removed by heating the sample at 120 °C in a convection oven for 3 minutes. After completely removing the solvent, the glass coupons were wiped with a clean, dry wipe that was identical to the wipe that was used to coat the polymers onto the glass. The dried samples were tested as described below.

**Table 7. Polymer-coated PCT glass substrates. Comparative examples, which do not comprise an organosilane pendant group, are designated with the notation "comparative".**

| Example No. | Polymer Designation | Polymer Example No. |
|---|---|---|
| 134 | p(DMAEMA-C16Br/A-174/IOA) | 6 |
| 135 comparative | p(DMAEMA-C 16Br/AA/IOA) | 7 |
| 136 | p(DMAEMA-C 16Br/A-174/NHMAc/IOA) | 8 |
| 137 comparative | p(DMAEMA-C 16Br/HFPOMA/EOMA/AA) | 15 |
| 138 comparative | p(DMAEMA-C 16Br/HFPOMA/HEMA/AA) | 16 |
| 139 | p(DMAEMA-C16Br/A-174/IOA) | 6 |
| 140 comparative | p(DMAEMA-C16Br/AA/IOA) | 7 |
| 141 | p(DMAEMA-C16Br/A-174/NHMAc/IOA) | 8 |
| 142 comparative | p(DMAEMA-C16Br/HFPOMA/EOMA/AA) | 15 |
| 143 comparative | p(DMAEMA-C 16Br/HFPOMA/HEMA/AA) | 16 |

### EXAMPLES 144-155

### Method of coating hybrid antimicrobial polymers onto projected capacitive touch (PCT) sensor glass substrates

AEM5700 antimicrobial solution was obtained from Aegis Environmental (Midland, Michigan). The AEM5700 was diluted to 1 wt% into IPA to make the working solution. PCT glass coupons were prepared as described in Examples 134-143. Polymer solutions from the Examples shown in Table 2 were diluted in IPA to 5 wt%. The diluted polymer solutions were mixed 1:1 with the working solution of AEM5700. The resulting mixtures (shown in Table 8) were applied to the glass coupons and the hybrid antimicrobial polymer-coated coupons were treated, dried, and cleaned as described in Examples 134-143 and were tested as described below.

Controls (Examples 149 and 155) consisted of applying 5 milliliters of the AEM5700 working solution directly to a glass coupon and treating, cleaning, and drying the coupon as described for Examples 134-143.

**Table 8. Hybrid antimicrobial polymer-coated PCT glass substrates. Comparative examples, which do not comprise an organosilane pendant group, are designated with the notation "comparative".**

| Example No. | Polymer Designation | Polymer Example No. |
|---|---|---|
| 144 | P(DMAEMA-C16Br/A-174/IOA/AEM) | 6 |
| 145 comparative | p(DMAEMA-C16Br/AA/IOA/AEM) | 7 |
| 146 | p(DMAEMA-C16Br/A-174/NHMAc/IOA/AEM) | 8 |
| 147 comparative | p(DMAEMA-C16Br/HFPOMA/EOMA/AA/AEM) | 15 |
| 148 comparative | p(DMAEMA-C16Br/HFPOMA/HEMA/AA/AEM) | 16 |
| 149 | No Polymer - AEM5700 Control | - |
| 150 | p(DMAEMA-C16Br/A-174/IOA/AEM) | 6 |
| 151 comparative | p(DMAEMA-C 16Br/AA/IOA/AEM) | 7 |
| 152 | p(DMAEMA-C16Br/A-174/NHMAc/IOA/AEM) | 8 |
| 153 comparative | p(DMAEMA-C16Br/HFPOMA/EOMA/AA/AEM) | 15 |
| 154 comparative | p(DMAEMA-C16Br/HFPOMA/HEMA/AA/AEM) | 16 |
| 155 | No Polymer - AEM5700 Control | - |

### EXAMPLE 156

### Physical testing for antimicrobial polymer-coated glass substrates and hybrid antimicrobial polymer-coated glass substrates.

ASTM test methods are published by ASTM International (West Conshohocken, PA). The coated glass substrates were tested for a variety of physical properties. The hydrophobicity of the polymer-coated surface was tested by measuring the contact angle of a drop of deionized water using the ASTM D7334.7606 test method. The scratch resistance of the polymer-coated surface was tested using the ASTM D7027-05 test ("Scratch test") with a constant load of 1000 g using Moh's hardness pens. The result is reported as the hardest pen that did not cause a scratch with the 1000 g load. The transmitted haze and transmittance of the polymer-coated glass substrates were measured using the ASTM D1003 on the Haze Gard Plus meter, from BYK-Gardner GmbH, Geretsried, Germany, and transmission is reported as the percent of light transmitted through the sample. The clarity was measured using a BYK-Gardner Haze Gard Plus instrument, calibrated using 0% (Black cover) standard and a clarity standard of 77.8% provided by BYK-Garner (catalog # 4732). Reflected Haze is measured according to test method ASTM E430. Gloss at 20° and 60° was measured on a BYK Gloss meter using ASTM D523 test method.

The eraser rub test, which is a measurement of the durability of the coating, was performed according to the United States Military Specification for the Coating of Optical Glass Elements (MIL-C-675C) dated 22 August 1980. The value reported is the number of eraser rubs required to remove the coating from the glass.

Tables 9-11 show the results of screening a variety of antimicrobial polymer and hybrid antimicrobial polymer compositions for their physical properties on each respective type of glass substrate.

**Table 9. Physical properties of antimicrobial-coated SCT glass substrates. "NR" denotes that the specified data were not collected. All data reported in the table are the average of ten independent determinations of the physical characteristic for each coupon of coated glass. The data for Example 86 is the average (and standard deviation) for ten independent determinations of the physical characteristic for ten separate coupons of glass that were coated on separate days with the same working solution of AEM5700. Comparative examples, which do not comprise an organosilane pendant group, are designated with the notation "comp.".**

| Coated Substrate (Example No.) | Contact Angle | Scratch Test | %T | % Haze | Clarity | Ref Haze | 20° gloss | 60° gloss |
|---|---|---|---|---|---|---|---|---|
| 40 | 89.56 | NR | 91.20 | 8.70 | 89.60 | 475.00 | 70.20 | 81.50 |
| 41 | 58.85 | NR | 92.00 | 6.30 | 89.80 | 413.00 | 73.00 | 83.60 |
| 42 | 88.45 | NR | 91.80 | 5.80 | 88.50 | 431.00 | 75.50 | 85.50 |
| 43 comp. | 108.25 | NR | 91.80 | 8.90 | 88.50 | 418.70 | 66.80 | 77.80 |
| 44 | 87.54 | Pencil # 7 | 91.00 | 7.44 | 88.97 | 454.00 | 76.90 | 83.90 |
| 45 comp. | 85.46 | Pencil # 7 | 91.00 | 6.57 | 87.70 | 441.00 | 80.17 | 86.00 |
| 46 | 84.85 | Pencil # 7 | 90.77 | 6.73 | 87.03 | 426.00 | 80.57 | 86.03 |
| 47 comp. | 80.72 | Pencil # 7 | 90.97 | 6.68 | 87.27 | 451.00 | 79.03 | 85.10 |
| 48 comp. | 72.06 | Pencil # 7 | 90.87 | 7.06 | 88.60 | 440.00 | 78.10 | 85.90 |
| 49 comp. | 76.59 | Pencil # 7 | 90.93 | 7.51 | 88.47 | 446.00 | 76.30 | 82.80 |
| 50 comp. | 53.17 | Pencil # 7 | 91.73 | 7.56 | 88.23 | 439.67 | 71.87 | 83.33 |
| 51 comp. | 67.11 | Pencil # 6 | 91.77 | 7.28 | 88.17 | 429.67 | 71.97 | 83.80 |
| 52 comp. | 53.04 | Pencil # 6 | 91.73 | 8.29 | 89.33 | 423.67 | 68.07 | 77.67 |
| 53 comp. | 53.34 | Pencil # 6 | 91.77 | 7.83 | 88.20 | 433.67 | 66.13 | 74.53 |
| 54 comp. | 49.67 | Pencil # 6 | 91.63 | 7.66 | 88.90 | 430.33 | 71.90 | 81.47 |
| 55 comp. | 49.24 | Pencil # 7 | 91.60 | 8.08 | 89.13 | 432.00 | 71.57 | 79.03 |
| 56 comp. | 58.68 | Pencil # 7 | 91.73 | 6.71 | 89.33 | 437.33 | 71.90 | 83.93 |
| 57 comp. | 58.68 | Pencil # 7 | 91.77 | 6.35 | 91.03 | 401.33 | 78.80 | 86.73 |
| 58 comp. | 58.84 | Pencil # 8 | 91.57 | 12.57 | 89.37 | 393.00 | 66.03 | 69.83 |
| 59 | 67.26 | Pencil # 7 | 91.30 | 10.08 | 89.53 | 416.33 | 70.80 | 78.37 |
| 60 | 63.31 | Pencil # 7 | 91.50 | 9.26 | 89.63 | 423.00 | 71.13 | 80.17 |
| 61 | 67.98 | Pencil # 6 | 91.80 | 6.62 | 89.57 | 435.33 | 73.87 | 84.40 |
| 62 | 72.61 | Pencil # 7 | 91.80 | 6.41 | 89.80 | 436.67 | 74.50 | 85.03 |
| 63 | 85.00 | NR | 90.80 | 6.98 | 89.30 | 477.00 | 72.30 | 87.80 |
| 64 | 75.00 | NR | 91.90 | 5.80 | 87.80 | 440.00 | 74.50 | 85.80 |
| 65 | 89.00 | NR | 91.90 | 6.10 | 89.50 | 418.00 | 73.10 | 84.60 |
| 66 comp. | 81.00 | NR | 91.80 | 6.80 | 89.20 | 422.00 | 70.80 | 83.80 |
| 67 | 88.16 | Pencil # 7 | 91.10 | 6.76 | 87.80 | 460.00 | 77.97 | 84.93 |
| 68 comp. | 88.24 | Pencil # 7 | 90.90 | 7.39 | 88.90 | 455.00 | 78.87 | 85.73 |
| 69 | 86.81 | Pencil # 7 | 91.03 | 7.04 | 87.60 | 420.00 | 79.03 | 86.70 |
| 70 comp. | 85.48 | Pencil # 7 | 90.97 | 7.56 | 88.70 | 453.00 | 75.30 | 83.53 |
| 71 comp. | 75.26 | Pencil # 7 | 90.87 | 7.08 | 88.10 | 438.00 | 78.10 | 85.23 |
| 72 comp. | 81.08 | Pencil # 7 | 90.97 | 8.31 | 88.80 | 451.00 | 76.27 | 82.17 |
| 73 comp. | 67.17 | Pencil # 7 | 91.57 | 7.68 | 89.57 | 421.00 | 71.17 | 80.47 |
| 74 comp. | 77.47 | Pencil # 8 | 91.70 | 7.03 | 89.43 | 432.00 | 76.27 | 87.67 |
| 75 comp. | 65.68 | Pencil # 6 | 91.63 | 7.28 | 88.20 | 434.33 | 76.83 | 86.70 |
| 76 comp. | 67.11 | Pencil # 7 | 91.80 | 7.49 | 88.50 | 463.00 | 73.60 | 84.30 |
| 77 comp. | 66.72 | Pencil # 6 | 91.63 | 8.36 | 89.17 | 437.33 | 74.17 | 84.50 |
| 78 comp. | 71.04 | Pencil # 7 | 91.33 | 7.95 | 88.57 | 439.67 | 75.07 | 86.13 |
| 79 comp. | 64.77 | Pencil # 7 | 91.77 | 7.35 | 89.63 | 428.00 | 75.43 | 86.13 |
| 80 comp. | 75.25 | Pencil # 7 | 91.53 | 6.74 | 90.00 | 435.00 | 78.50 | 88.27 |
| 81 comp. | 70.76 | Pencil # 7 | 91.70 | 8.95 | 89.50 | 407.67 | 70.27 | 76.57 |
| 82 | 62.54 | Pencil # 5 | 91.60 | 9.10 | 90.20 | 413.33 | 70.90 | 76.77 |
| 83 | 67.52 | Pencil # 6 | 91.53 | 8.20 | 90.50 | 404.33 | 72.13 | 79.93 |
| 84 | 69.72 | Pencil # 6 | 91.90 | 6.29 | 90.53 | 418.33 | 75.60 | 85.07 |
| 85 | 69.79 | Pencil # 6 | 91.87 | 6.40 | 90.73 | 417.67 | 75.37 | 84.50 |
| 86 comp. | 81.02±10.50 | Pencil # 7 | 91.63±0.35 | 7.00±0.93 | 89.20±0.60 | 431.40±19.40 | 74.70±2.96 | 84.60±2.77 |

**Table 10. Physical properties of antimicrobial-coated DST glass substrates. All data reported in the table are the average of ten independent determinations of the physical characteristic for each coupon of coated glass. The data for Example 133 is the average (and standard deviation) for ten independent determinations of the physical characteristic for ten separate coupons of glass that were coated on separate days with the same working solution of AEM5700. Comparative examples, which do not comprise an organosilane pendant group, are designated with the notation "comp.".**

| **Coated Substrate (Example No.)** | **Contact Angle** | **Scratch Test with hardness pens loaded 1000g** | **%T** | **% Haze** | **Clarity** | **Ref Haze** | **20° gloss** | **60° gloss** |
|---|---|---|---|---|---|---|---|---|
| 87 | 85.21 | pencil 6 visible | 92.3 | 4.79 | 78.3 | 564 | 22.4 | 75.4 |
| 88 | 67.42 | pencil 6 visible | 92.3 | 4.52 | 77.9 | 578 | 22 | 75.4 |
| 89 | 89.7 | pencil 7 visible | 92.4 | 4.55 | 77.47 | 571 | 21 | 74.4 |
| 90 comp. | 83.79 | pencil 8 visible | 92 | 10.77 | 77.3 | 513 | 17.5 | 58.23 |
| 91 | 94.09 | Pencil #5 visible | 91.9 | 10.45 | 72.9 | 487 | 14.7 | 55.4 |
| 92 comp. | 82.26 | Pencil #5 visible | 92.2 | 5.6 | 74.1 | 533.7 | 17.1 | 68.2 |
| 93 | 88.13 | Pencil #5 visible | 92.1 | 5.8 | 73.2 | 532 | 16.7 | 67.6 |
| 94 comp. | 76.39 | Pencil #4 visible | 92.2 | 5.8 | 73 | 529.7 | 16.7 | 67.7 |
| 95 comp. | 79.14 | Pencil #4 visible | 92 | 6.1 | 72.9 | 528.7 | 16.7 | 67.3 |
| 96 comp. | 83.58 | Pencil #4 visible | 91.8 | 9.8 | 72.6 | 493.3 | 14.8 | 58.1 |
| 97 comp. | 58.81 | Pencil# 7-Visible | 92.30 | 5.91 | 76.90 | 547.33 | 19.13 | 70.80 |
| 98 comp. | 67.00 | Pencil# 7-Visible | 92.33 | 5.66 | 77.63 | 560.00 | 20.17 | 73.20 |
| 99 comp. | 71.33 | Pencil# 8-Visible | 92.43 | 6.87 | 78.73 | 508.33 | 17.90 | 68.43 |
| 100 comp. | 58.56 | Pencil# 7 visible | 92.17 | 6.83 | 71.60 | 520.67 | 15.70 | 65.97 |
| 101 comp. | 64.58 | Pencil# 8-Visible | 92.30 | 6.77 | 74.83 | 489.33 | 17.47 | 65.63 |
| 102 comp. | 60.30 | Pencil# 7 visible | 92.13 | 7.72 | 72.73 | 477.33 | 14.67 | 62.37 |
| 103 comp. | 54.51 | Pencil# 7 visible | 92.10 | 6.17 | 74.90 | 551.33 | 18.70 | 69.77 |
| 104 comp. | 62.70 | Pencil# 7 visible | 92.13 | 6.23 | 74.67 | 549.67 | 18.27 | 68.67 |
| 105 comp. | 67.55 | Pencil# 8-Visible | 91.67 | 15.00 | 72.47 | 486.33 | 15.13 | 51.60 |
| 106 | 61.35 | Pencil# 6 visible | 92.47 | 9.31 | 74.57 | 506.00 | 16.43 | 59.03 |
| 107 | 54.38 | Pencil# 6 visible | 92.50 | 6.69 | 76.95 | 537.50 | 18.00 | 66.55 |
| 108 | 44.63 | Pencil #7 visible | 92.67 | 5.51 | 75.70 | 536.67 | 17.53 | 68.33 |
| 109 | 58.18 | Pencil #7 visible | 92.53 | 8.12 | 74.73 | 503.00 | 15.60 | 58.90 |
| 110 | 88.88 | pencil 7 visible | 92.1 | 10.33 | 67.3 | 470 | 13.1 | 56 |
| 111 | 64.24 | pencil 7 visible | 92.3 | 5.98 | 69.7 | 518 | 15.2 | 65.3 |
| 112 | 90.44 | pencil 7 visible | 92.3 | 5.93 | 70.1 | 520 | 15.6 | 65.9 |
| 113 comp. | 86.34 | pencil 7 visible | 92 | 9.67 | 70.6 | 500 | 14.8 | 60.3 |
| 114 | 94.46 | Pencil #4 visible | 92.10 | 6.92 | 72.97 | 516.00 | 16.10 | 63.80 |
| 115 comp. | 89.31 | Pencil #4 visible | 92.10 | 6.17 | 72.93 | 522.67 | 16.23 | 66.30 |
| 116 | 89.77 | Pencil #4 visible | 92.10 | 5.94 | 71.40 | 523.67 | 15.87 | 66.37 |
| 117 comp. | 84.88 | Pencil #6 visible | 92.07 | 6.19 | 70.37 | 513.00 | 15.20 | 65.07 |
| 118 comp. | 82.01 | Pencil #6 visible | 92.03 | 6.16 | 71.83 | 520.67 | 16.00 | 66.67 |
| 119 comp. | 86.04 | Pencil #7 visible | 92.00 | 6.62 | 76.13 | 541.00 | 17.80 | 67.07 |
| 120 comp. | 69.72 | Pencil# 6-Visible | 92.40 | 6.17 | 78.50 | 536.00 | 20.90 | 71.13 |
| 121 comp. | 94.94 | Pencil# 5-Visible | 92.63 | 4.49 | 77.37 | 558.67 | 21.87 | 74.53 |
| 122 comp. | 96.19 | Pencil# 5-Visible | 92.47 | 5.22 | 78.47 | 539.00 | 20.07 | 71.23 |
| 123 comp. | 69.78 | Pencil# 6 visible | 92.13 | 5.22 | 74.20 | 540.33 | 18.40 | 69.53 |
| 124 comp. | 66.84 | Pencil# 7 visible | 92.10 | 5.37 | 73.67 | 547.33 | 18.20 | 69.20 |
| 125 comp. | 67.10 | Pencil# 7 visible | 91.97 | 5.35 | 73.77 | 551.00 | 18.03 | 69.70 |
| 126 comp. | 68.12 | Pencil# 7 visible | 92.00 | 5.62 | 74.27 | 546.00 | 17.50 | 68.17 |
| 127 comp. | 66.43 | Pencil# 7 visible | 92.03 | 5.52 | 74.13 | 554.33 | 18.77 | 70.43 |
| 128 comp. | 70.32 | Pencil# 7 visible | 91.97 | 6.45 | 74.10 | 549.33 | 18.17 | 66.80 |
| 129 | 56.55 | Pencil #7 visible | 92.40 | 10.17 | 75.27 | 516.00 | 16.67 | 59.87 |
| 130 | 52.83 | Pencil #7 visible | 92.43 | 8.36 | 74.90 | 512.67 | 16.27 | 62.00 |
| 131 | 73.09 | Pencil #6 visible | 92.57 | 7.02 | 72.87 | 514.67 | 15.37 | 62.03 |
| 132 | 65.94 | Pencil #6 visible | 92.57 | 6.73 | 71.83 | 526.33 | 15.90 | 63.97 |
| 133 comp. | 75.29±18.64 | Pencil #6 visible | 92.32±0.21 | 5.43±0.48 | 73.30±2.87 | 537.80±19.25 | 17.23±1.78 | 68.48±3.08 |

**Table 11. Physical properties of antimicrobial-coated PCT glass substrates. All data reported in the table are the average of ten independent determinations of the physical characteristic for each coupon of coated glass. The data for Examples 149 and 150 are the averages (and standard deviations) for ten independent determinations of the physical characteristic for two separate coupons of glass that were coated on separate days with the same working solution of AEM5700. Comparative examples, which do not comprise an organosilane pendant group, are designated with the notation "comp.".**

| **Coated Substrate (Example No.)** | **Advancing Contact Angle** | **Eraser Rub Test** | **%T** | **% Haze** | **Clarity** | **Ref Haze** | **20° gloss** | **60° gloss** |
|---|---|---|---|---|---|---|---|---|
| 134 | 98.38 | 18.50 | 93.60 | 5.75 | 91.43 | 468.33 | 62.67 | 78.07 |
| 135 comp. | 92.69 | 27.00 | 93.27 | 5.74 | 91.23 | 482.67 | 67.10 | 80.10 |
| 136 | 82.05 | 10.00 | 93.57 | 6.33 | 90.93 | 465.67 | 59.87 | 74.90 |
| 137 comp. | 77.65 | 16.00 | 93.1 | 7.3 | 90.3 | 483.3 | 61.2 | 73.6 |
| 138 comp. | 94.60 | 15.50 | 93.6 | 5.0 | 92.7 | 461.3 | 67.1 | 81.8 |
| 139 | 88.72 | 13.50 | 93.2 | 5.4 | 92.7 | 471.7 | 70.6 | 82.7 |
| 140 comp. | 86.10 | 12.50 | 93.5 | 5.9 | 92.0 | 440.0 | 64.9 | 79.0 |
| 141 | 80.52 | 15.50 | 93.2 | 5.2 | 91.8 | 477.3 | 68.9 | 82.7 |
| 142 comp. | 86.89 | 17.50 | 93.6 | 5.3 | 93.2 | 453.0 | 68.7 | 82.7 |
| 143 comp. | 80.81 | 15.50 | 93.2 | 5.4 | 92.2 | 475.7 | 71.2 | 83.7 |
| 144 | 94.09 | 20.00 | 93.53 | 5.69 | 93.17 | 446.33 | 66.90 | 80.93 |
| 145 comp. | 93.71 | 37.50 | 93.20 | 5.17 | 91.77 | 483.00 | 70.40 | 83.73 |
| 146 | 87.01 | 15.00 | 92.90 | 4.34 | 90.47 | 518.67 | 68.17 | 79.60 |
| 147 comp. | 85.82 | 26.50 | 93.20 | 5.47 | 92.23 | 473.33 | 69.90 | 82.37 |
| 148 comp. | 95.14 | 27.50 | 93.50 | 4.79 | 92.30 | 471.67 | 67.73 | 84.03 |
| 150 | 92.55 | 36.50 | 93.07 | 6.35 | 90.97 | 475.67 | 65.53 | 77.90 |
| 151 comp. | 86.94 | 15.50 | 92.9 | 4.9 | 88.4 | 526.7 | 62.6 | 74.7 |
| 152 | 84.87 | 22.50 | 93.1 | 6.8 | 90.3 | 478.0 | 63.4 | 75.5 |
| 153 comp. | 84.96 | 15.00 | 92.9 | 4.6 | 88.2 | 529.7 | 62.9 | 75.1 |
| 154 comp. | 87.33 | 21.50 | 93.2 | 6.7 | 91.3 | 468.0 | 65.5 | 78.1 |
| 149 | 88.34±1.39 | 25.50±4.24 | 93.2±0.6 | 5.0±0.1 | 90.6±1.7 | 526.05.2± | 64.2±0.9 | 78.1±1.1 |
| 155 | 90.62±2.43 | 36.00±6.36 | 93.3±0.0 | 4.8±0.1 | 92.2±1.3 | 452.4±22.1 | 71.9±1.3 | 85.6±2.0 |

### EXAMPLE 157

### Antimicrobial activity testing for antimicrobial polymer-coated glass substrates and hybrid antimicrobial polymer-coated glass substrates.

Antimicrobial activity of the coated glass substrates was tested using three standard methods. The ASTM 2149 test method (ASTM International; West Conshohocken, PA) was used to evaluate the effectiveness of the non-leaching antimicrobial polymer-coated surfaces of the glass coupons. Overnight cultures of *Staphylococcus aureus* (ATCC 6538) and *Staphylococcus. epidermidis* (ATCC 12228) were diluted in phosphate buffer to a concentration of approximately 1x10⁶ CFU/ml. A 1x1-inch (2.54 cm x 2.54 cm) square of glass sample was placed into a tube containing 50ml of dilute bacterial suspensions. The samples were incubated with constant agitation for 24 hours at 28+/-1° C. Immediately after the samples were placed into the tubes (T= 0 hr) and after 24 hours (T=24 hr), a small aliquot of the suspension was serially-diluted and the diluted samples were inoculated onto Petrifilm Aerobic Count (AC) Plates (3M Company, St. Paul, MN) according to the manufacturer's instructions. The plates were incubated for 48 hours at 35°C±1°C. Bacterial colonies from appropriate dilution were counted according to the manufacturer's instructions and recorded as colony-forming units (CFU) per ml.

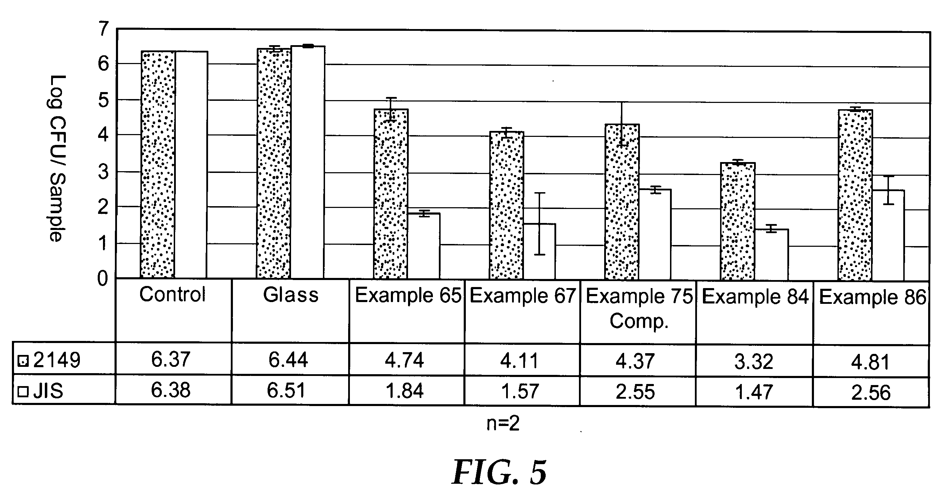

The JIS Z 2801 test method (Japan Industrial Standards; Japanese Standards Association; Tokyo, JP) was used to evaluate the antibacterial activity of antibacterial polymer-coated glass substrates. The bacterial inoculum was prepared in a solution of 1 part Nutrient Broth (NB) and 499 parts phosphate buffer. A portion of the inoculum was used to determine the number of viable bacteria in the inoculum. Another portion of the bacterial suspension (150 µL) was placed onto the surface of the glass sample and the inoculated glass sample was incubated for the specified contact time at 28+/-1°C (see Figure 5). After incubation, the glass sample was placed into 20ml of D/E Neutralizing Broth. The number of surviving bacteria in the Neutralizing broth was determined by inoculating the broth onto nutrient agar using a Spiral Plater WASP II, DW Scientific, Shipley, West Yorkshire, UK, incubating the plates for 24 hours at 35°C±1°C and counting the colonies using a colony reader (ProtoCol colony Counter; Microbiology International; Frederick, MD). FIG. 6 shows a comparison of the results from 5 different coated samples that were tested using the ASTM 2149 test method and the JIS Z 2801 test method. This experimental data from the JIS test method showed a greater than 3-log reduction of the microorganisms after a two hour contact time with the antimicrobial polymer-treated glass samples. In this experiment, the control was SCT glass that was not treated with an antimicrobial polymer.

Test method ASTM E2180-01 was designed to evaluate quantitatively the antimicrobial effectiveness of antimicrobial coatings on the coated surfaces. Overnight cultures of *Staphylococcus aureus* (ATCC 6538) were used to inoculate the agar slurry, which was applied to the antimicrobial polymer-coated surface of the glass samples. The microorganisms were recovered from inoculated agar slurry using Dey/Engley (D/E) Neutralizing Broth. Bacterial plate counts were performed using 3M Petrifilm Aerobic Count (AC) Plates (3M Company, St. Paul, MN, USA) according to the manufacturer's instructions. The colony counts were recorded as colony-forming unit (CFU) per cm². The difference between bacterial count in a slurry when the inoculum is immediately applied to the surface (T=0 hr) and the bacterial count in the slurry after 24 hours of contact with the antimicrobial surface is recorded as the log₁₀ reduction. The polymer-treated glass samples were compared to an untreated sample of PET film (Acrylate-primer coated polyethylene terephthalate film, 4 mil (0.10 mm) thick, obtained from Mitsubishi Polyester Film, Greer, SC). The results (not shown) indicate that each of the antimicrobial polymer-treated glass samples had a log₁₀ reduction of about 0.5 to about 0.8 using this test method.

### COMPARATIVE EXAMPLE 158

### Synthesis of comparative antimicrobial polymer

A comparative antimicrobial polymer was synthesized according to Example No. 2 of PCT Patent Application Publication No. WO2010/036465. The polymer was diluted in IPA to 5 wt% and was coated onto conductively-coated glass (part number 29617), obtained from Pilkington North America, Inc. (Toledo, OH), as described in Examples 40-62 above. The surface hydrophobicity of the resulting coated substrate was compared to the coated sample of Example 65 by measuring the contact angle of a drop of deionized water on each coated substrate using ASTM test method D7334.7606. The results are shown in Table 12. The results indicate that the antimicrobial polymer of Example 65 was more hydrophobic than the polymer of comparative Example 158.

**Table 12. Comparison of surface hydrophobicity of coated substrates.**

| **Coated Substrate** | **Advancing Contact Angle** |
|---|---|
| Comparative Example 158 | 30.6 |
| Example 65 | 96.12 |

### EXAMPLES 159-162

### Synthesis of comparative antimicrobial polymer

Samples of SCT glass were prepared and coated using the procedures described in Examples 40-62. Samples of PCT glass were prepared and coated using the procedures described in Examples 134-143. The respective coating formulations are listed in Table 13. A1120 (N-(beta-aminoethyl)-gamma-aminopropyltrimethoxysilane) was added to the coating solutions as an adhesion-promoting reagent. Tin-octoate was added to the coating solutions as a catalyst to promote cross-linking reactions. After applying the coating formulations to the glass and allowing evaporation of the solvent, the coated substrates in Examples 159, 161, and 162 were placed in a convection oven (138° C) for 4 minutes and then cooled to room temperature. After applying the coating formulations to the glass and allowing evaporation of the solvent, the coated substrates in Example 160 was placed in a convection oven (120° C) for 3 minutes and then cooled to room temperature. After cooling, all substrates were immediately washed in a Billco washer as described in Examples 40-62.

**Table 13. Coating solutions for Examples 159-160.**

| **Example** | Substrate | Coating Formulation |
|---|---|---|
| 159 | SCT | A 1:1 blend of 3 Wt% (in IPA) AEM5700 and 5 Wt% (in IPA) of the polymer of Example 6, to which 2 Wt% tin-octoate and 0.04 Wt% A1120 were added. |
| 160 | PCT | A 1:1 blend of 3 Wt% (in IPA) AEM5700 and 5 Wt% (in IPA) of the polymer of Example 6, to which 2 Wt% tin-octoate and 0.04 Wt% A1120 were added. |
| 161 | SCT | 5 Wt% (in IPA) of the polymer of Example 6, to which 2 Wt% tin-octoate was added |
| 162 | PCT | 5 Wt% (in IPA) of the polymer of Example 6, to which 2 Wt% tin-octoate was added |

The samples were tested for antimicrobial activity using the JIS Z 2801 test procedure described in Example 157. Uncoated samples of each type of glass were used as controls. The bacterial inoculum was prepared in a solution of 1 part Nutrient Broth (NB) and 499 parts phosphate buffer. A portion of the inoculum was used to determine the number of viable bacteria in the inoculum. Another portion of the bacterial suspension (150 µL) was placed onto the surface of the glass sample and the inoculated glass sample was incubated for the specified contact time at 28°±1°C. After incubation, the glass sample was placed into 20 ml of D/E Neutralizing Broth. The number of surviving bacteria in the Neutralizing broth was determined by inoculating the broth onto nutrient agar using a Spiral Plater WASP, incubating the plates for 24 hours at 35°C±1°C and counting the colonies using a colony reader (ProtoCol colony Counter; Microbiology International; Frederick, MD). The results are shown in Tables 14 and 15, which show data from experiments conducted on separate days.

### COMPARATIVE EXAMPLES 163-164

### Synthesis of comparative antimicrobial polymer

Samples of SCT glass were prepared and coated using the procedures described in Examples 40-62. Samples of PCT glass were prepared and coated using the procedures described in Examples 134-143. The respective coating formulations are listed in Table 13. A1120 (N-(beta-aminoethyl)-gamma-aminopropyltrimethoxysilane) was added to the coating solutions as an adhesion-promoting reagent. Tin-octoate was added to the coating solutions as a catalyst to promote cross-linking reactions. After applying the coating formulations to the glass and allowing evaporation of the solvent, the coated substrates in Comparative Example 161 was placed in a convection oven (138° C) for 4 minutes and then cooled to room temperature. After applying the coating formulations to the glass and allowing evaporation of the solvent, the coated substrates in Comparative Example 162 was placed in a convection oven (120° C) for 3 minutes and then cooled to room temperature. After cooling, all substrates were immediately washed in a Billco washer as described in Examples 40-62.

**Table 13. Coating solutions for Comparative Example 163-164.**

| **Comparative Example** | Substrate | Coating Formulation |
|---|---|---|
| 163 | SCT | A mixture of 3Wt% AEM5700 in IPA with 2 Wt% tin-octoate and 0.04% A1120. |
| 164 | PCT | A mixture of 3Wt% AEM5700 in IPA with 2 Wt% tin-octoate and 0.04% A1120. |

The samples were tested for antimicrobial activity using the JIS Z 2801 test procedure described for Examples 159-160. Uncoated samples of each type of glass were used as controls. The results are shown in Tables 14 and 15, which show data from experiments conducted on separate days.

**Table 14. Tests for antibacterial properties of coated-glass samples. Bacterial suspensions were tested for viable cells after 15 minutes and 2 hours contact time with the coated glass surfaces, respectively. All results are the average of three samples tested for each type. Cfu = colony-forming unit.**

| Sample | Log₁₀ cfu (15-min) | Log₁₀ cfu (2 hr) | Log₁₀ Reduction of viable bacteria after 15-min. contact time | Log₁₀ Reduction of viable bacteria after 2-hour contact time |
|---|---|---|---|---|
| Control (SCT glass) | 5.56 | 5.58 | 0 | 0 |
| Control (PCT glass) | 5.53 | 5.65 | 0 | 0 |
| Example 159 | 3.95 | 1.48 | 1.61 | 4.1 |
| Example 160 | 3.44 | 1.05 | 2.09 | 4.6 |
| Comp. Example 163 | 5.37 | 4.27 | 0.19 | 1.31 |
| Comp. Example 164 | 5.42 | 4.12 | 0.11 | 1.53 |

**Table 15. Tests for antibacterial properties of coated-glass samples. Bacterial suspensions were tested for viable cells after 15 minutes and 2 hours contact time with the coated glass surfaces, respectively. All results are the average of three samples tested for each type. Cfu = colony-forming unit.**

| Sample | Log₁₀ cfu (15-min) | Log₁₀ cfu (2 hr) | Log₁₀ Reduction of viable bacteria after 15-min. contact time | Log₁₀ Reduction of viable bacteria after 2-hour contact time |
|---|---|---|---|---|
| Control (SCT glass) | 5.51 | 5.64 | 0 | 0 |
| Control (PCT glass) | 5.53 | 5.65 | 0 | 0 |
| Example 159 | 3.38 | 0.90 | 2.13 | 4.61 |
| Example 160 | 4.32 | 1.07 | 1.2 | 4.43 |
| Example 161 | 4.35 | 0.99 | 1.16 | 4.52 |
| Example 162 | 4.48 | 1.45 | 1.04 | 4.05 |
| Comp. Example 163 | 5.14 | 3.69 | 0.37 | 1.82 |
| Comp. Example 164 | 5.37 | 4.21 | 0.15 | 1.29 |

The present invention has now been described with reference to several specific embodiments foreseen by the inventor for which enabling descriptions are available. Insubstantial modifications of the invention, including modifications not presently foreseen, may nonetheless constitute equivalents thereto. Thus, the scope of the present invention should not be limited by the details and structures described herein, but rather solely by the following claims, and equivalents thereto.

## Claims

1. A composition, comprising:
a solvent;
a polymer having a plurality of pendant groups comprising,
a first pendant group comprising a first quaternary ammonium component,
a second pendant group comprising a nonpolar component, and
a third pendant group comprising a first organosilane component; and an adhesion-promoting reagent.

2. The composition of claim 1, wherein the adhesion-promoting reagent is selected from the group consisting of 3-triethoxysilyl-N-(1,3-dimethyl-butyliden)propylamine, N-phenyl-3-aminopropyltrimethoxysilane, and 3-[2-(2-aminoethylamino)ethylamino]propyl-trimethoxysilane, 3-aminopropyltrimethoxysilane, 3-aminopropyltriethoxysilane, 3-(2-aminoethyl)aminopropyltrimethoxysilane, (aminoethylaminomethyl)phenethyltrimethoxysilane, (aminoethylaminomethyl) phenethyltriethoxysilane, N-(2-aminoethyl)-3-aminopropylmethyldimethoxysilane, bis-(γ-triethoxysilylpropyl) amine, N-(2-aminoethyl)-3-aminopropyltributoxysilane, 6-(aminohexylaminopropyl)trimethoxysilane, 4-aminobutyltrimethoxysilane, 4-aminobutyltriethoxysilane, p-(2-aminoethyl) phenyltrimethoxysilane, 3-aminopropyltris(methoxyethoxyethoxy)silane, 3 -aminopropylmethyldiethoxysilane, tetraethoxysilane and oligomers thereof, methyltriethoxysilane and oligomers thereof, an oligomeric aminosilane, 6, 3-(N-methylamino)propyltrimethoxysilane, N-(2-aminoethyl)-3-aminopropylmethyldimethoxysilane, N -(2-aminoethyl)-3-aminopropylmethyldiethoxysilane, N -(2-aminoethyl)-3-aminopropyltrimethoxysilane, N -(2-aminoethyl)-3-aminopropyltriethoxysilane, 3-aminopropylmethyldiethoxysilane, 3-aminopropylmethyldimethoxysilane, 3-aminopropyldimethylmethoxysilane, and 3-aminopropyldimethylethoxysilane.

3. The composition of claim 1, further comprising a second quaternary ammonium component.

4. The composition of claim 2 or claim 3, further comprising a second organosilane component.

5. An article made by the process of:
forming a first composition of an organic polymer in organic solvent, the polymer having a plurality of pendant groups comprising,
a first pendant group comprising a first quaternary ammonium component,
a second pendant group comprising a nonpolar component, and
a third pendant group comprising a first organosilane component;
mixing a second quaternary ammonium component with the first composition to form a first mixture; and
contacting the first mixture with a substrate under conditions suitable to form covalent linkages between the organic polymer, the substrate, and the second quaternary ammonium component.

## Patentansprüche

1. Zusammensetzung, umfassend:
ein Lösungsmittel;
ein Polymer mit einer Vielzahl von anhängenden Gruppen, umfassend:
eine erste anhängende Gruppe umfassend einen ersten quartären Ammoniumbestandteil,
eine zweite anhängende Gruppe umfassend einen unpolaren Bestandteil, und
eine dritte anhängende Gruppe umfassend einen ersten Organosilanbestandteil und ein adhesionsförderndes Reagenz.

2. Zusammensetzung nach Anspruch 1, wobei das adhesionsfördernde Reagenz ausgewählt ist aus der Gruppe, bestehend aus 3-Triethoxysilyl-N-(1,3-di-methyl-butyliden)propylamin, N-Phenyl-3-aminopropyltrimethoxysilan und 3-[2-(2-Aminoethylamino)ethylamino]propyltrimethoxysilan, 3-Aminopropyltrimethoxy-silan, 3-Aminopropyltriethoxysilan, 3-(2- Aminoethyl)aminopropyltrimethoxysilan, (Aminoethylaminomethyl)phenethyltrimethoxysilan, (Aminoethylaminomethyl)-phenethyltriethoxysilan, N-(2-Aminoethyl)-3-aminopropylmethyldimethoxysilan, bis-(γ-Triethoxysilylpropyl)amin, N-(2-Aminoethyl)-3-aminopropyltributoxysilan, 6-(Aminohexylaminopropyl)trimethoxysilan, 4-Aminobutyltrimethoxysilan, 4-Amino-butyltriethoxysilan, p-(2-Aminoethyl)phenyltrimethoxysilan, 3-Aminopropyltris(methoxyethoxyethoxy)silan, 3-Aminopropylmethyldiethoxysilan, Tetraethoxysilan und Oligomere davon, Methyltriethoxysilan und Oligomere davon, ein oligomeres Aminosilan, 6, 3-(N-Methylamino)propyltrimethoxysilan, N-(2-Aminoethyl)-3-aminopropylmethyldimethoxysilan, N-(2-Aminoethyl)-3-aminopropylmethyldiethoxy-silan, N-(2-Aminoethyl)-3-aminopropyltrimethoxysilan, N-(2-Aminoethyl)-3-amino-propyltriethoxysilan, 3-Aminopropylmethyldiethoxysilan, 3-Aminopropylmethyldimethoxysilan, 3-Aminopropyldimethylmethoxysilan und 3-Aminopropyldimethyl-ethoxysilan.

3. Zusammensetzung nach Anspruch 1, ferner umfassend einen zweiten quartären Ammoniumbestandteil.

4. Zusammensetzung nach Anspruch 2 oder Anspruch 3, ferner umfassend einen zweiten Organosilanbestandteil.

5. Artikel, hergestellt durch folgendes Verfahren:
Bilden einer ersten Zusammensetzung eines organischen Polymers in organischem Lösungsmittel, wobei das Polymer eine Vielzahl von anhängenden Gruppen aufweist, umfassend:
eine erste anhängende Gruppe umfassend einen ersten quartären Ammoniumbestandteil,
eine zweite anhängende Gruppe umfassend einen unpolaren Bestandteil, und
eine dritte anhängende Gruppe umfassend einen ersten quartären Organosilanbestandteil;
Mischen eines zweiten quartären Ammoniumbestandteils mit der ersten Zusammensetzung, um eine erste Mischung zu bilden, und
Inkontaktbringen der ersten Mischung mit einem Substrat unter Bedingungen, die geeignet sind, kovalente Bindungen zwischen dem organischen Polymer, dem Substrat und dem zweiten quartären Ammoniumbestandteil zu bilden.

## Revendications

1. Composition comprenant :
un solvant ;
un polymère comportant une pluralité de groupes pendants comprenant,
un premier groupe pendant comprenant un premier composant ammonium quaternaire,
un deuxième groupe pendant comprenant un composant apolaire, et
un troisième groupe pendant comprenant un premier composant organosilane ; et un réactif promoteur d'adhésion.

2. Composition selon la revendication 1, dans laquelle le réactif promoteur d'adhésion est choisi dans le groupe constitué de 3-triéthoxysily-N-(1,3-diméthyl-butylidène)propylamine, N-phényl-3-aminopropyltriméthoxysilane, et 3-[2-(2-aminoéthylamino)éthylamino]propyl-triméthoxysilane, 3-aminopropyltriméthoxysilane, 3-aminopropyltriéthoxysilane, 3-(2-aminoéthyl)aminopropyltriméthoxysilane, (aminoéthylaminométhyl)phénéthyltriméthoxysilane, (aminoéthylaminométhyl) phénéthyltriéthoxysilane, N-(2-aminoéthyl)-3-aminopropylméthyldiméthoxysilane, bis-(γ-triéthoxysilylpropyl)amine, N-(2-aminoéthyl)-3-aminopropyltributoxysilane, 6-(aminohexylaminopropyl)triméthoxysilane, 4-aminobutyltriméthoxysilane, 4-aminobutyltriéthoxysilane, p-(2-aminoéthyl)phényltriméthoxysilane, 3-aminopropyltris(méthoxyéthoxyéthoxy)silane, 3-aminopropylméthyldiéthoxysilane, tétraéthoxysilane et oligomères de celui-ci, méthyltriéthoxysilane et oligomères de celui-ci, un aminosilane oligomère, 6,3-(N-méthylamino)propyltriméthoxysilane, N-(2-aminoéthyl)-3-aminopropylméthyldiméthoxysilane, N-(2-aminoéthyl)-3-aminopropylméthyldiéthoxysilane, N-(2-aminoéthyl)-3-aminopropyltriméthoxysilane, N-(2-aminoéthyl)-3-aminopropyltriéthoxysilane, 3-aminopropylméthyldiéthoxysilane, 3-aminopropylméthyldiméthoxysilane, 3-aminopropyldiméthylméthoxysilane et 3-aminopropyldiméthyléthoxysilane.

3. Composition selon la revendication 1, comprenant en outre un deuxième composant ammonium quaternaire.

4. Composition selon la revendication 2 ou la revendication 3, comprenant en outre un deuxième composant organosilane.

5. Article fabriqué par le procédé consistant à :
former une première composition d'un polymère organique dans un solvant organique, le polymère comportant une pluralité de groupes pendants comprenant,
un premier groupe pendant comprenant un premier composant ammonium quaternaire,
un deuxième groupe pendant comprenant un composant apolaire, et
un troisième groupe pendant comprenant un premier composant organosilane ;
mélanger un deuxième composant ammonium quaternaire avec la première composition pour former un premier mélange ; et
mettre en contact le premier mélange avec un substrat dans des conditions appropriées pour former des liaisons covalentes entre le polymère organique, le substrat et le deuxième composant ammonium quaternaire.
